# EUROPEAN PATENT APPLICATION

(11) **EP 3 988 580 A2**
(43) Date of publication of application: **27.04.2022**
(21) Application number: 20826379.8
(22) Date of filing: 19.05.2020
(51) Int. Cl.: C08B 37/16, C08G 79/025, C08L 5/16, C08L 85/02, A61L 27/52, A61L 27/54, A61L 27/38, A61K 47/69, A61K 47/58

(54) **HYDROGEL INCLUSION COMPLEX CONTAINING BIOACTIVE SUBSTANCE BOUND TO TEMPERATURE-SENSITIVE POLYPHOSPHAZENE BY HOST-GUEST INTERACTION THROUGH BETA-CYCLODEXTRIN, AND USE THEREOF**

(30) Priority: 19.06.2019 KR 20190073071
(71) Applicant: Korea Institute of Science and Technology, Seoul 02792 (KR)
(72) Inventor: SONG, Soo Chang, Seoul 02792 (KR); HONG, Ki Hyun, Seoul 02792 (KR)
(74) Representative: Gilani, Anwar
(86) International application number: PCT/KR2020/006548
(87) International publication number: WO 2020/256287

(57) **Abstract**

Provided is a hydrogel composition including thermosensitive poly(phosphazene) to which a plurality of hydrophobic amino acids, hydrophilic polymers, and host molecules are substituted; and a physiologically active material linked to a guest molecule, wherein the poly(phosphazene) and the physiologically active material form a conjugate by inclusion of the guest molecule in the host molecule via a host-guest interaction.

When injected into a living body, the hydrogel composition of the present disclosure slowly releases the physiologically active material and provides favorable conditions for stem cell differentiation. In addition, the hydrogel composition, in which the kind, ratio, and sequence of the physiologically active material are controlled to be suitable for stem cell differentiation, may be prepared with high reproducibility.

## Description

### BACKGROUND OF THE INVENTION

### 1. Field of the Invention

The present invention relates to a hydrogel inclusion complex bound with a physiologically active material by a host-guest interaction through beta-cyclodextrin substituted to thermosensitive poly(phosphazene), and use thereof.

### 2. Description of the Related Art

As a biocompatible material to construct neotissues for the purpose of tissue regeneration, numerous kinds of materials based on a bioconjugation technology were utilized such as nanoparticle, decellularized matrix, and hydrogel. Among these materials, hydrogel is an excellent material source for tissue engineering owing to capacities in absorption of huge amounts of water, high biocompatibility, and living tissue resemblance. In detail, synthetic hydrogels such as poly(lactic acid-glycolic acid) derivatives, poly(lactic acid) derivatives, hyaluronic acid, polyurethane acrylates, and many other kinds of biomedical polymers are used for tissue regeneration by employing both physiologically active materials and stem cells.

Efforts have been made to fine-tune biocompatible materials such that they are implanted in a living body and tissue regeneration occurs in a desired direction. The fine-tuned biocompatible materials connote well-controlled materials in both physical and chemical aspects. This fine tuning of biocompatible materials is strongly required in several therapeutic perspectives, for example, an enhanced therapeutic window in drug delivery system, immunotherapy efficacy (to avoid auto-immune response or immunotoxicity), disease specific targeting, and appropriate stem cell stimulations for regenerative engineering. Generally, physical control of biocompatible materials means the mechanical property control and microstructure control, affecting cell characteristics or therapeutic drug release patterns. Chemical control of biocompatible materials means the control of chemical linkage. Specifically, chemical linkage between two molecules is called bio-conjugation (Kalia J, Raines RT. Advances in Bioconjugation., Current organic chemistry14, 138-147 (2010)). Although the bio-conjugation technology offers advantages to biomaterials, including structural stabilization from chemical degradation, an improved body residence time, and reduction of immunogenicity, there still remain issues such as toxicity issues, low reproducibility, and complex synthesis processes. In particular, since high chemoselectivity is resulted from the employment of acrylate, methacrylate, copper (I)-catalyzed azide alkyne cycloaddition (CuAAC), and free radicals, there is a high risk issue for toxicity owing to remnants of such groups (Spicer CD, Pashuck ET, and Stevens MM, Achieving Controlled Biomolecule-Biomaterial Conjugation, Chemical reviews, 118, 7702-7743 (2018)). Also, the low reproducibility prevents the fine tuning of biocompatible materials.

In particular, attempts have been made to control the differentiation of stem cells using hydrogels including physiologically active materials. There are diverse bioactive molecules for proliferation and differentiation of MSCs (mesenchymal stem cells), such as small chemicals, proteins, and peptides. Since administration of MSCs alone makes their differentiation into a desired lineage and their survival in a harsh body environment difficult, the assistance with such hydrogels and bioactive molecules is essential to the ideal tissue regeneration. Recently, various approaches for the tissue regeneration by employing MSCs were explored with several kinds of proteins for the desired MSC differentiation. However, even through physical and chemical controls of stem cells, bioactive molecules, and hydrogels, fine tuning of stem cell differentiation has still not been achieved to a satisfactory level.

Meanwhile, a host-guest interaction forms a non-covalent complex of two or more molecules by unique structural recognition through a molecular self-recognition system. There are many macrocyclic host molecules such as cyclodextrins (CDs), crown ethers, cyclophanes, cryptands, and curcubiturils. Among these host molecules, cyclodextrin is suitable for the tissue engineering or biological applications owing to its low toxicity and low immunogenicity. Famous cyclodextrins mainly utilized in the industrial and research realm are α-, β-, and γ-CD. Most of all, β-CD is a superb molecule owing to its high water solubility post-modification, excellent inner cavity size for guest molecule loading, and relatively low cost. The host-guest interaction using β-CD provides several benefits to biocompatible materials involving a facile supramolecular structure building, and an avoidance of multiple synthesis steps and a complicated purification process during the fabrication process. Over the past few decades, β-CD has been actually employed in various biomedical applications such as drug delivery systems, diagnostic applications, and tissue engineering.

However, there have been no attempts to introduce a physiologically active material using the host-guest interaction into a hydrogel such as thermosensitive poly(phosphazene). Under this circumstance, the present inventors had attempted to introduce the host-guest interaction with the purpose of regulating many kinds of bioactive factors in 3D hydrogel of poly(organophosphazene) bearing β-CD. PPZ has been developed as a biocompatible and thermosensitive hydrogel by the present inventors over the past few decades (see Korean Patent Application Publication Nos. 10-2005-0012533 A and 10-2008-0110472 A, etc.), and therapeutically valuable drugs, such as chemical drugs, proteins, and genes, can be delivered by the same.

The present inventors succeeded in the synthesis of β-CD PPZ, and have studied the stoichiometric control of bioactive guest molecules of β-CD PPZ to overcome the limitations of bio-conjugation systems. They formed various functional guest molecules yielding hydrogels without a further synthesis batch of β-CD PPZ through a diverse combination of the guest molecule ratios in the host, and found that had, fine-tuned differentiation can be achieved by application of these hydrogels, thereby completing the present invention.

### SUMMARY OF THE INVENTION

An object of the present invention is to provide a hydrogel inclusion complex including thermosensitive poly(phosphazene) to which a plurality of hydrophobic amino acids, hydrophilic polymers, and beta-cyclodextrin (β-cyclodextrin; β-CD) as a host molecule are substituted; and a physiologically active material linked directly or via a linker to one or more molecules, as a guest molecule, selected from the group consisting of adamantine, azobenzene, cholesterol, tert-butyl, cyclohexyl ester, and naphthyl, wherein the guest molecule is conjugated to all or part of the beta-cyclodextrin by inclusion of the guest molecule into the beta-cyclodextrin via a host-guest interaction.

Another object of the present invention is to provide a hydrogel composition for regulating stem cell differentiation, which includes as active ingredients, thermosensitive poly(phosphazene) to which hydrophobic amino acids, hydrophilic polymers, and beta-cyclodextrin are substituted; and a stem cell differentiation regulator linked directly or via a linker to one or more molecules, as a guest molecule, selected from the group consisting of adamantine, azobenzene, cholesterol, tert-butyl, cyclohexyl ester, and naphthyl.

Still another object of the present invention is to provide a method of regenerating a tissue, the method including a step of injecting, into a damaged tissue site, a hydrogel composition including, as active ingredients, thermosensitive poly(phosphazene) to which a plurality of hydrophobic amino acids, hydrophilic polymers, and beta-cyclodextrin are substituted; and a stem cell differentiation regulator linked directly or via a linker to one or more molecules, as a guest molecule, selected from the group consisting of adamantine, azobenzene, cholesterol, tert-butyl, cyclohexyl ester, and naphthyl.

Still another object of the present invention is to provide a hydrogel composition for inhibiting cancer cell proliferation or metastasis, which includes, as active ingredients, thermosensitive poly(phosphazene) to which a plurality of hydrophobic amino acids, hydrophilic polymers, and beta-cyclodextrin are substituted; and IL-2 linked directly or via a linker to one or more molecules, as a guest molecule, selected from the group consisting of adamantine, azobenzene, cholesterol, tert-butyl, cyclohexyl ester, and naphthyl.

Still another object of the present invention is to provide thermosensitive poly(phosphazene) to which a plurality of hydrophobic amino acids, hydrophilic polymers, and beta-cyclodextrin are substituted.

### BRIEF DESCRIPTION OF THE DRAWINGS

FIG. 1 shows a schematic illustration of β-CD PPZ synthesis.
FIG. 2 shows a chemical structure of β-CD PPZ and characterization thereof, wherein FIG. 2A shows a chemical structure of β-CD-conjugated acid PPZ, FIG. 2B shows a schematic illustration of β-CD PPZ including isoleucine, PEG, and β-CD, FIG. 2C shows ¹H NMR results of β-CD PPZ and acid PPZ, and FIG. 2D shows FT-IR of β-CD-conjugated PPZ, as compared to a mixture of acid PPZ, β-CD, and acid PPZ.
FIG. 3 shows a schematic illustration of an Ad-RGD synthesis process.
FIG. 4 shows NMR results of Ad-PEG-NH₂.
FIG. 5 shows FT-IR results of Ad-PEG-NH₂.
FIG. 6 shows NMR results of Ad-PEG-MeAc.
FIG. 7 shows NMR results of Ad-PEG-RGD.
FIG. 8A shows 2D-NOESY NMR results of β-CD PPZ and Ad-RGD, FIG. 8B shows the exact proton positions of β-CD and adamantine, and FIG. 8C shows 2D-NOESY integration values for cross peaks of β-CD PPZ and Ad-RGD-mixed hydrogel according to various Ad-RGD concentrations (β-CD:Ad-RGD=100:0-100:100).
FIG. 9 shows gelation properties of β-CD PPZ and β-CD PPZ/Ad-RGD mixture, wherein FIG. 9A shows viscosity of acid PPZ (black) and β-CD PPZ (red), FIG. 9B shows the occurrence of gelation in body temperature, and FIG. 9C shows details of thermosensitive gelation property of acid PPZ and β-CD PPZ.
FIG. 10 shows results of survival rates of MSCs verified by a live/dead assay and CCK-8, wherein FIG. 10A shows results of a live/dead assay when a combination of β-CD PPZ and Ad-RGD of 0 mL to 100 mL was applied to MSCs (green represents live MSCs and red represents dead MSCs) (measurements were made at time points of day 0, day 1, day 3, and day 7), FIG. 10B shows illustration of MSCs cultured in ECM mimicking 3D hydrogel, and FIG. 10C shows a comparison result of survival rates of MSCs using CCK-8 after 7 days.
FIG. 11 shows relative gene expression levels (normalized to β-actin) for 7 days in MSC normal growth media in which various concentrations of Ad-RGD is added, wherein FIGS. 11A and 11D each show relative gene expression levels of ALP and collagen I (markers for osteogenic factors), FIGS. 11B and 11E each show relative gene expression levels of collagen II and aggrecan (markers for chondrogenic factors), and FIGS. 11C and 11F each show relative gene expression levels of C/EBPα and PPARγ (markers for adipogenic factor).
FIG. 12 shows a schematic illustration of MSC differentiation activity of the conjugate of β-CD PPZ and Ad-RGD, based on the MSC differentiation control activity of the hydrogel composition of Example 1.
FIG. 13 shows a schematic illustration of the Ad-TGF/Ad-HAV synthesis process in a hydrogel composition of Example 2.
FIG. 14 shows NMR results of Ad-TGF.
FIG. 15 shows NMR results of Ad-HAV.

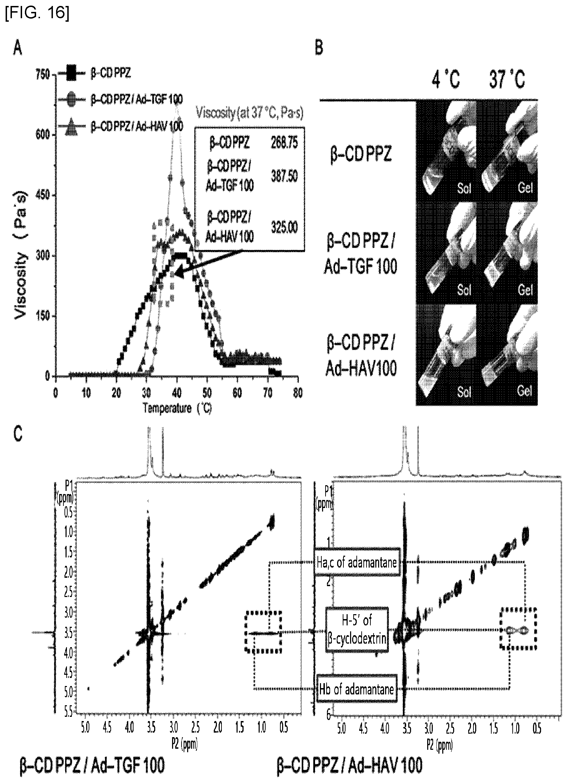

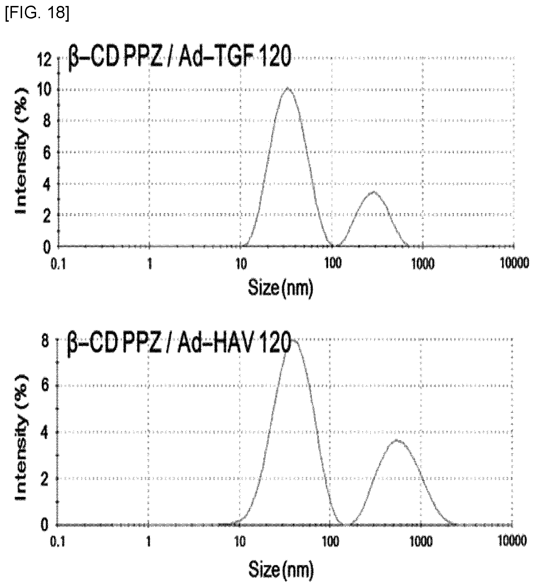

FIG. 16 shows the thermosensitive property of the β-CD PPZ/Ad-TGF or HAV conjugate via a host-guest interaction, wherein FIG. 18A shows thermosensitive gelation details for β-CD PPZ, β-CD PPZ/Ad-TGF 100, and β-CD PPZ/Ad-HAV 100 (viscosities at the body temperature (37°C) for each group were elucidated), FIG. 18B shows visualization of sol-gel transition for β-CD PPZ (top), β-CD PPZ/Ad-TGF 100 (middle), and β-CD PPZ/Ad-HAV 100 (bottom), and FIG. 18C shows 2D NOESY results for the evidence of a host-guest interaction between β-CD PPZ and Ad-TGF (left) and 2D NOESY results for the evidence of a host-guest interaction between PPZ and Ad-HAV (right).

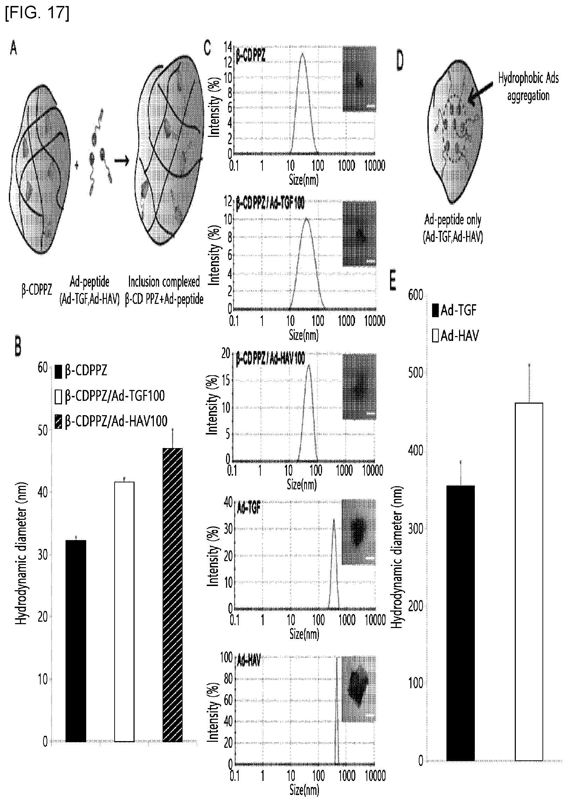

FIG. 17A shows a schematic illustration for a host-guest interaction between β-CD PPZ and Ad-peptide, FIG. 17B shows results of hydrodynamic diameters of β-CD PPZ, β-CD PPZ/Ad-TGF, and Ad-HAV (n=3), FIG. 17C shows size distribution results and TEM images of β-CD PPZ, β-CD PPZ/Ad-TGF, Ad-HAV, and Ad-peptides alone (Ad-TGF and Ad-HAV) (Scale bar represents 50 nm for β-CD PPZ, β-CD PPZ/Ad-TGF, and Ad-HAV, and 500 nm for Ad-peptides alone, respectively), FIG. 17D shows an illustration for aggregation of Ad-peptides alone in an aqueous state, and FIG. 17E shows results of hydrodynamic diameter of Ad-peptides alone.
FIG. 18 shows size distribution results of β-CD PPZ/Ad-TGF 120 and β-CD PPZ/Ad-HAV 120.
FIG. 19 shows host-guest interaction maintenance dependent on the Ad-peptide ratio gradient using an IVIS system, wherein FIG. 17A shows pictures for host-guest interactions in which Ad-TGF and Ad-HAV interacted in mice with β-CD PPZ and MSC tagged with FITC and rhodamine (Rho), respectively (in the period of 21 days, each of Ad-TGF and Ad-HAV gradient fluorescence was detected in the IVIS system), and FIG. 17B shows the average radient efficiency values of Ad-TGF (top) and Ad-HAV (bottom) for 21 days.

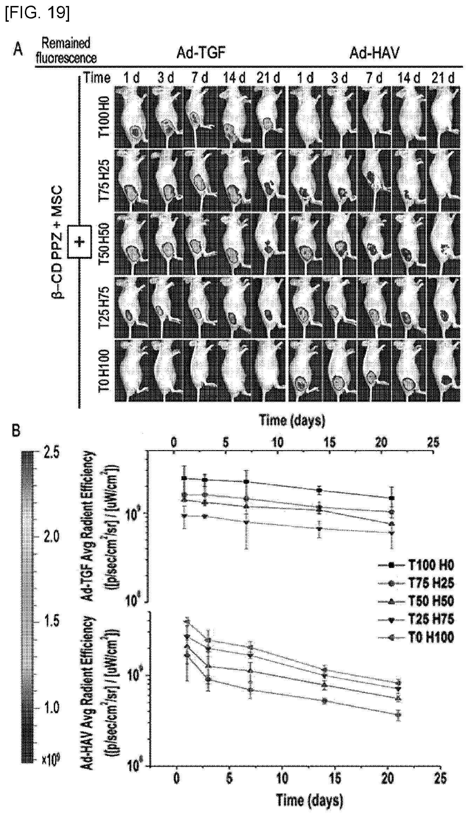

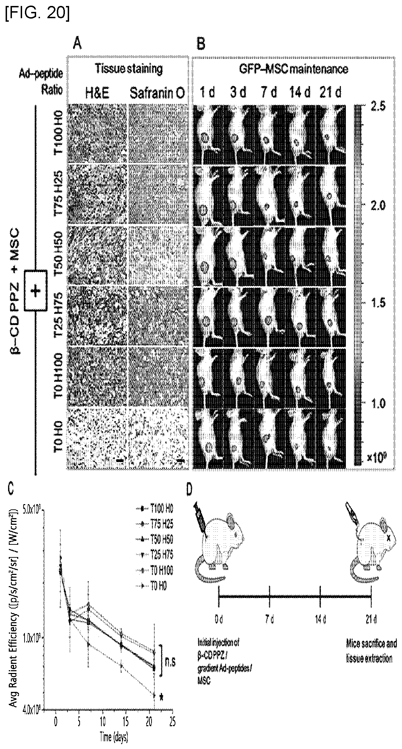

FIG. 20 shows tissue compatibility of the complex of β-CD PPZ/Ad-peptide/MSC of various concentrations, and its basic differentiation capacity, wherein FIG. 19A shows the results of H&E staining for the tissue compatibility verification of MSCs (left) and safranin-O staining for the demonstration of chondrogenic differentiation (Scale bar represents 100 µm), FIG. 19B shows the maintenance of stem cells in the conjugate of β-CD PPZ with Ad-peptides of various concentrations, measured using IVIS system, FIG. 19C shows the average radient efficiency values in mice of GFP tagged MSCs for 21 days (n=3), and FIG. 19D shows the basic illustration for an *in vivo* experimental schedule.
FIG. 21 shows MSC chondrogenic induction activity of β-CD/Ad-peptide of Example 2 (mice were sacrificed on 21st day of the post-injection), wherein FIG. 20A shows the results of immunohistochemistry with the aggrecan, which is a representative protein for chondrogensis detection (Scale bar represents 20 µm), FIG. 20B shows analysis results of Agg fluorescence intensities (n=3), and FIG. 20C shows Agg gene expression levels in the mice tissues (n=3).
FIG. 22 shows MSC chondrogenic induction activity of β-CD/Ad-peptide of Example 2 (mice were sacrificed on 21st day of the post-injection), wherein FIG. 21A shows the results of immunohistochemistry with the Col II, which is a representative protein for chondrogensis detection (Scale bar=20 µm), FIG. 20B shows analysis results of Col II fluorescence intensities (n=3), and FIG. 20C shows Col II gene expression levels in mice tissues (n=3).
FIG. 23 shows that β-CD/Ad-peptide of Example 2 did not induce MSC osteogenesis, wherein FIG. 22A shows results of Von Kossa staining with various Ad-peptide/β-CD PPZ/MSC (Scale bar represents 100 µm), and FIG. 22B shows gene expression levels of Runx2, which is a typical gene for osteogenesis (n=3).

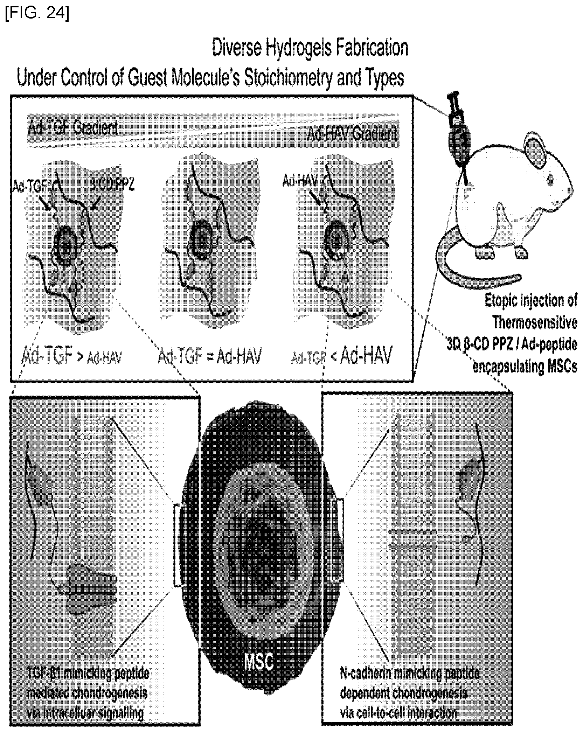

FIG. 24 shows a schematic illustration for the administration of mesenchymal stem cells (MSCs) treated with the hydrogel composition of Example 2, wherein in order to control the MSC fate to be suitable for chondrogenesis, TGF-β1 peptide and N cadherin peptide bound to the guest molecule could be smoothly introduced with stoichiometric flexibility in the hydrogel composition.

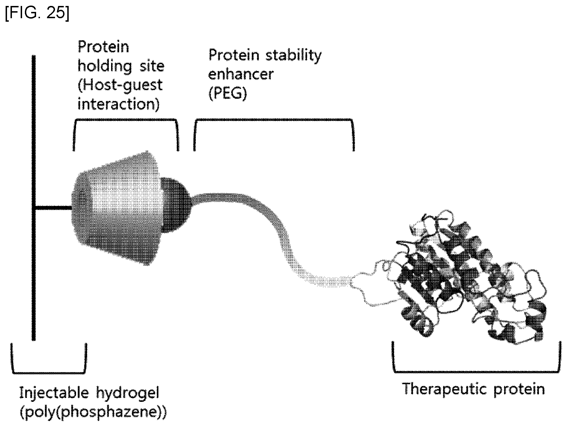

FIG. 25 shows a schematic illustration of use of a therapeutic protein such as a hydrogel composition of the Example 3 as a physiologically active material.
FIG. 26 shows a schematic illustration of Ad-IL2 formation via click chemistry.
FIG. 27 shows a schematic illustration of Ad-IL2 formation via EDC chemistry.

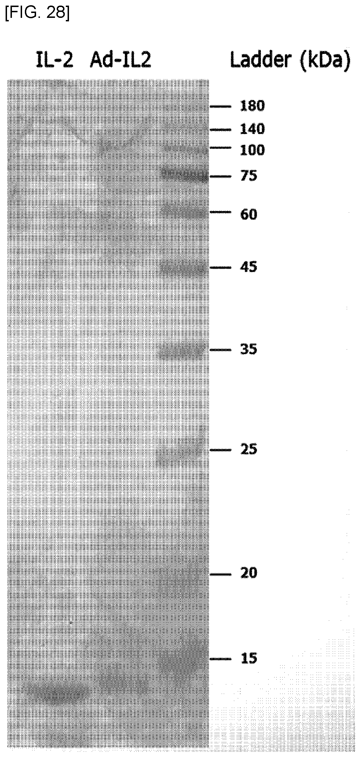

FIG. 28 shows the result of electrophoresis to examine formation of Ad-IL2 by conjugating a thiol group of the protein via amine-polyethylene glycol-adamantine.

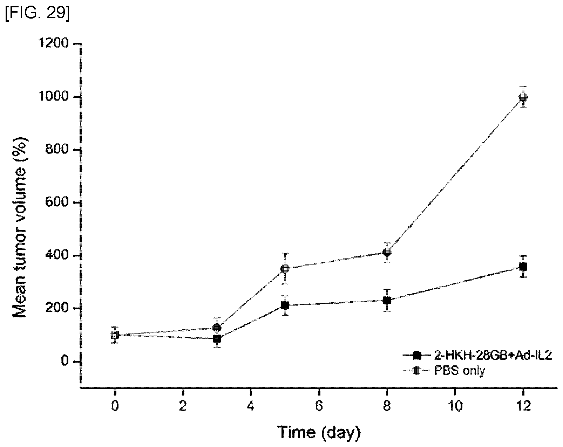

FIG. 29 shows tumor growth-inhibitory effects of Ad-IL2 chemically conjugated with adamantine.

### DETAILED DESCRIPTION OF THE PREFERRED EMBODIMENTS

Hereinafter, the present invention will be described in more detail.

Meanwhile, each description and embodiment disclosed herein may also be applied to other descriptions and embodiments. That is, all combinations of various elements disclosed herein fall within the scope of the present invention. Further, the scope of the present invention is not limited by the specific description described below.

Further, those skilled in the art will recognize, or be able to ascertain, using no more than routine experimentation, many equivalents to the specific embodiments of the invention described herein. Further, these equivalents should be interpreted to fall within the present invention.

To achieve the above objects, one aspect of the present invention provides a hydrogel inclusion complex including thermosensitive poly(phosphazene) to which a plurality of hydrophobic amino acids, hydrophilic polymers, and beta-cyclodextrin (β-cydodextrin; β-CD) as a host molecule are substituted; and a physiologically active material linked directly or via a linker to one or more molecules, as a guest molecule, selected from the group consisting of adamantine, azobenzene, cholesterol, tert-butyl, cyclohexyl ester, and naphthyl, wherein the guest molecule is conjugated to all or part of the beta-cyclodextrin by inclusion of the guest molecule into the beta-cyclodextrin via a host-guest interaction.

As used herein, the 'hydrogel' refers to a three-dimensional network structure formed from a polymer present in an aqueous solution, and is usually divided into hydrogels formed by chemical crosslinking via covalent bonds and hydrogels formed by physical crosslinking via physical interactions between molecules. The hydrogel of the present disclosure generally has a basic skeleton of thermosensitive poly(phosphazene), to which a host molecule is substituted, and forms a conjugate with a guest molecule, which is involved in a host-guest interaction with the host molecule and to which a physiologically active material is linked. The hydrogel composition is a biocompatible material injectable into a living body.

As used herein, 'thermosensitive' means a property that an aqueous polymer solution maintains a liquid state (sol) at a low temperature, but the aqueous polymer solution is transformed to a gel as the temperature increases. Specifically, it means a property of maintaining a liquid state at room temperature, while being transformed into a gel state at a temperature of 35°C to 37°C or higher.

Since the hydrogel of the present disclosure is introduced into the body to form a gel of a three-dimensional structure by body temperature, there are advantages in that it is easy to inject owing to the liquid state, and at the same time, a drug can be slowly released owing to its transition to a gel state. Further, the hydrogel in the gel state may form a niche (microenvironment) suitable for stem cell differentiation. For example, when stem cells are simply injected, they non-specifically spread throughout the body. In contrast, when stem cells are injected in a state mixed with the gel, it not only simply makes the injection easy, such as targeting a desired site thereby allowing the stem cells to be delivered to the desired site and to be retained therein, but also contributes to local delivery to a desired site.

Meanwhile, stem cell is characterized by having i) self-renewal and ii) pluripotency, regulating differentiation of the stem cell is an important factor, especially in tissue regeneration. Further, stem cell differentiation can be regulated by treating stem cells with the hydrogel composition including a stem cell differentiation regulator according to the present invention. In this regard, by regulating the type or ratio of the stem cell differentiation regulator, or both of them, stemness of the stem cells can be maintained, or the stem cells may be regulated to be differentiated to a specific state.

Furthermore, the hydrogel composition of the present invention can be used for tissue regeneration. As described above, since the hydrogel composition including a stem cell differentiation regulator of the present invention can regulate stem cell differentiation, it is possible to regulate stem cell differentiation at the corresponding site and promote regeneration of a desired tissue by injecting the composition to a damaged tissue site. In this regard, the composition may further include stem cells, but is not limited thereto, and the hydrogel composition itself may exert the effect of promoting tissue regeneration.

As used herein, the 'poly(phosphazene)' is substituted with hydrophobic amino acids and hydrophilic polymers such that it exhibits a thermosensitive property. Specifically, a hydrophobic amino acid ester and hydrophilic methoxypolyethylene glycol having a molecular weight in the range of 350 to 2,500 may be introduced into a linear polymer of dichlorophosphazene, and an amino acid, a peptide, or depsipeptide ester capable of controlling a degradation rate of the polymer may be partially introduced into the polymer. Further, functional groups may be introduced into the phosphazene-based polymer of the present invention, e.g., by directly introducing a substituent with functional groups such as a hydroxyl group, an amide group, an amino group, a thiol group, or a carboxyl group on the side chain into the main chain, or introducing the amino acid ester or peptide ester substituent, wherein the functional group is substituted with a protecting group, into the main chain of the polymer followed by removing the protecting group, or by a method of converting the polymer introduced with the substituent having the functional groups to another functional group. The thermosensitive poly(phosphazene) to be used in the present disclosure may be those known in the art (see Korean Patent Application Publication Nos. 20050012533A and 10-2008-0110472 A, etc.). In this regard, the thermosensitive poly(phosphazene) may include a plurality of hydrophobic amino acids, hydrophilic polymers, and beta-cyclodextrin at a molar ratio of (55 to 80) : (5 to 25) : (5 to 20), but is not limited thereto.

The hydrophobic amino acid may be any one or more selected from the group consisting of glycine, alanine, valine, leucine, isoleucine, methionine, proline, phenylalanine, and tryptophan. The hydrophobic amino acid has a structure of NHCH(R¹)CO₂R², wherein R¹ may be selected from the group consisting of H, CH₃, CH₂SH, CH(CH₃)₂, CH₂CH(CH₃)₂, CH(CH₃)C₂H₅, CH₂CH₂SCH₃, CH₂C₆H₅, CH₂C₆H₄OH, and CH₂C₂NH₂C₆H₄, and R² may be selected from the group consisting of H, CH₃, C₂H₅, C₃H₇, C₄H₉, CH₂C₆H₅, and CH₂CHCH₂.

The hydrophilic polymer may be polyalkylene glycol having a molecular weight in the range of 550 - 2,500, and specifically, polyethylene glycol, monomethoxy polyethylene glycol, or a block copolymer of ethylene glycol and propylene glycol. The number of the alkylene repeating unit of polyalkylene glycol may be in a range of 7 to 50.

The type or substituted amount of the hydrophobic amino acids and hydrophilic polymers, and the concentration of poly(phosphazene) and the like may be adjusted to control the gelling temperature, gel strength, and/or biodegradation rate of poly(phosphazene). For example, as the composition of the hydrophobic amino acid increases, the gelling temperature can be lowered, and as the concentration of poly(phosphazene) increases, the gelling temperature becomes lower, and the gel strength increases. Further, as the chain length of the hydrophilic polymer increases, the gel strength increases and the gelling temperature becomes higher.

The physiologically active material-bound hydrogel inclusion complex of the present disclosure may deliver, into a body, the physiologically active material which is linked to a guest molecule via 'host-guest interaction'. The 'host-guest interaction' forms a non-covalently bound complex of two or more molecules through unique structural recognition of a molecular self-recognition system. As compared with a conjugate system via covalent bonding, holding bioactive molecules through the host-guest interaction is more simple reproducible. According to Experimental Examples 1 to 4 of the present disclosure, it was found that a specific conjugate was produced in proportion to the content and/or ratio of the guest molecule prepared before mixing. As described, as a result of using the host-guest interaction, the content and/or ratio of the physiologically active material attached to the surface of poly(phosphazene) may be easily controlled. Furthermore, the physiologically active material linked to the guest molecule may be slowly released by cleavage of the linkage with the host molecule in the body.

As used herein, the term 'host molecule' refers to any material that is able to capture the guest molecule, and has a hydrophobic cavity and a hydrophilic surface. Specifically, the physiologically active material-bound hydrogel of the present disclosure includes beta-cyclodextrin as the host molecule. The beta-cyclodextrin is suitable for tissue engineering or biological applications owing to its low toxicity and low immunogenicity.

As used herein, the term 'guest molecule' refers to a molecule that is trapped in the cavity of the host molecule. Since the cavity of the host molecule shows hydrophobicity, the guest molecule also shows hydrophobicity, and a material having a size suitable for inclusion in the cavity may be used. For example, the hydrogel inclusion complex of the present disclosure includes beta-cyclodextrin as the host molecule, and thus it is preferable to includes, as the guest molecule, adamantine, azobenzene, cholesterol, tert-butyl, cyclohexyl ester, naphthyl, or a combination thereof, which can specifically interact with the host molecule. In this regard, the guest molecule may be further modified to promote the activity of the physiologically active material.

As used herein, the term 'physiologically active material' refers to a material exhibiting activity when injected into a body or a cell, and may be one or more selected from the group consisting of proteins, peptides, vaccines, genes, hormones, anti-cancer drugs, angiogenesis inhibitors, sugars, polyols, sugar-containing polyols, polymer-containing polyols, sugar-containing amino acids, and sugar-containing ions.

The proteins may be selected from the group consisting of exendin-4, erythropoietin (EPO), interferon-alpha, interferon-beta, interferon-gamma, growth hormone (human, pig, cow, etc.), growth hormone releasing factor, nerve growth factor (NGF), granulocyte-colony stimulating factor (G-CSF), granulocyte macrophage-colony stimulating factor (GM-CSF), macrophage-colony stimulating factor (M-CSF), blood clotting factor, insulin, oxytocin, vasopressin, adrenocorticotropic hormone, fibroblast growth factor (FGF), epidermal growth factor (EGF), platelet-derived growth factor (PDGF), insulin-like growth factor (IGF), vascular endothelial growth factor (VEGF), transforming growth factor-beta

(TGF-β), nerve growth factor, brain-derived neurotrophic factor (BDNF), neurotrophin-3 (NT-3), neurotrophin-4/5, prolactin, luliberin, luteinizing hormone releasing hormone (LHRH), LHRH agonists, LHRH antagonists, somatostatin, glucagon, interleukin-2 (IL-2), interleukin-11 (IL-11), gastrin, tetragastrin, pentagastrin, urogastrone, secretin, calcitonin, enkephalins, endorphins, angiotensins, thyrotropin releasing hormone (TRH), tumor necrosis factor (TNF), tumor necrosis factor related apoptosis inducing ligand (TRAIL), heparinase, bone morphogenic protein (BMP), human atrial natriuretic peptide (hANP), glucagon-like peptide (GLP-1), renin, bradykinin, bacitracins, polymyxins, colistins, tyrocidine, gramicidins, cyclosporins, neurotensin, tachykinin, neuropeptide Y (NPY), peptide YY (PYY), vasoactive intestinal polypeptide (VIP), and pituitary adenylate cyclase-activating polypeptide (PACAP).

The peptides may be biomimetic peptides derived from the natural proteins. For example, the peptides may be selected from the group consisting of collagen 1-derived GFOGER and DGEA; laminin-derived YIGSR, SIKVAV, IKVAV, IKLLI, LRGDN, and SINNNR; laminin γ1-derived LRE, PDGSR, GTFALRGDNGQ, CFALRGDNP, NPWHSIYITRFG, TWYKIAFQRNRK, KAFDITYVRLKF, and LGTIPG; fibronectin-derived GRGDS, PKRGDL, NGRAHA, GACRGDCLGA(cyclic), IDAPS, REDV, PHSRN, KQAGDV, LDV, WQPPRARI, SPPRRARV, LIGRKK, IWKHKGRDVILKKDVRFYC, KLDAPT, and PRARI; vitronectin-derived CKKQRFRHRNRKG; osteopotin-derived KRSR, FHRRIKA, CGGNGEPRGDTYRAY, SVVYGLR, and ELVTDFPTDLPAT; elastin-derived VPGIG and VGVAPG; collagen 4-derived MNYYSNS and CNYYSNS; thrombospondin-derived CSVTCG, GRGDAC, FQGVLQNVRFVF, AELDVP, and VALDEP; nidogen-1-derived GFRGDGQ and SIGFRGDGQTC; N-cadherin-derived HAV; and TGF-β1-derived FLPASGL, PWPLPYL, WGLLDLT, PAERLRS, RNLDGWS, NLSSSWI, TLPSNTH, MSAFPFL, SRLGQYI, PFGPLPP, TIASTLH, PRAPADV, and ESPLKRQ.

The vaccine may be one or more selected from the group consisting of hepatitis vaccine and the like.

The gene may be one or more selected from the group consisting of small interference RNA (siRNA), plasmid DNA, antisense oligodeoxynucleotide (AS-ODN), and the like.

The hormone may be one or more selected from the group consisting of testosterone, estradiol, progesterone, prostaglandins, and their synthetic analogs, and a substance that is modified or shows the same effect.

The anti-cancer drug may be one or more selected from the group consisting of paclitaxel, doxorubicin, 5-fluorouracil, cisplatin, carboplatin, oxaliplatin, tegafur, irinotecan, docetaxel, cyclophosphamide, cemcitabine, ifosfamide, mitomycin C, vincristine, etoposide, methotrexate, topotecan, tamoxifen, vinorelbine, camptothecin, danuorubicin, chlorambucil, bryostatin-1, calicheamicin, mayatansine, levamisole, DNA recombinant interferon alfa-2a, mitoxantrone, nimustine, interferon alfa-2a, doxifluridine, formestane, leuprolide acetate, megestrol acetate, carmofur, teniposide, bleomycin, carmustine, heptaplatin, exemestane, anastrozole, estramustine, capecitabine, goserelin acetate, polysaccharide potassium, medroxyprogesterone acetate, epirubicin, letrozole, pirarubicin, topotecan, altretamine, toremifene citrate, BCNU, taxotere, actinomycin D, anasterozole, belotecan, imatinib, floxuridine, gemcitabine, hydroxyurea, zoledronate, vincristine, flutamide, valrubicin, streptozocin, polyethylene glycol conjugated anti-cancer agent, and their synthetic analogs, and a substance that is modified or shows the same effect.

The angiogenesis inhibitor may be one or more selected from the group consisting of clodronate, 6-deoxy-6-demethyl-4-dedimethylaminotetracycline (COL-3), doxycycline, marimastat, 2-methoxyestradiol, squalamine, SU5164, thalidomide, TNP-470, combretastatin A4, soy isoflavone, enzastaurin, CC 5013 (Revimid; Celgene Corp, Warren, NJ), celecoxib, ZD 6474, halofuginone hydrobromide, interferon-alpha, bevacizumab, shark cartilage extract (AE-941), interleukin-12, vascular endothelial growth factor-trap (VEFG-trap), cetuximab, rebimastat, matrix metalloproteinase (MMPs) inhibitor (e.g., BMS-275291 (Bristol-Myers Squibb, New York, NY), S-3304), etc.), protein kinase C beta inhibitor (e.g., LY317615), endostatin, vatalanib (PTK787/ZK 222584), sunitinib malate (SU11248), cilengitide (EMD-121974), humanized monoclonal antibody MEDI-522, EOS-200-4, integrin alpha-5-beta-1 antagonist (ATN-161), and their synthetic analogs, and a substance that is modified or shows the same effect.

The sugars may be one or more selected from the group consisting of lactose, glucose, dextran, mannose, sucrose, trehalose, maltose, and ficoll.

The polyols may be one or more selected from the group consisting of innositol, mannitol, and sorbitol.

The sugar-containing polyols may be sucrose-mannitol, glucose-mannitol, or a combination thereof.

The polymer-containing polyols may be one or more selected from the group consisting of trehalose-polyethylene glycol (trehalose-PEG), sucrose-polyethylene glycol (sucrose-PEG), and sucrose-dextran.

The sugar-containing amino acids may be sorbitol-glycine, sucrose-glycine, or a combination thereof.

The sugar-containing ions may be trehalose-zinc sulfate (trehalose-ZnSO₄), maltose-zinc sulfate (maltose-ZnSO₄), or a combination thereof.

The physiologically active material may be a stem cell differentiation regulator. In this case, the hydrogel composition may be used in controlling stem cell differentiation when co-cultured with stem cells. The stem cell differentiation regulator is the same as described below.

Alternatively, the physiologically active material may be a material that exhibits a therapeutic effect in a body, but is not limited thereto.

In the hydrogel inclusion complex of the present disclosure, the physiologically active material may be connected via a linker. The 'linker' (or spacer) provides a gap between the guest molecule and the physiologically active material, making it easy for the physiologically active material of the 3D hydrogel to adhere to a bioactive site. For example, as the linker between the guest molecule and the physiologically active material, polyethylene glycol (PEG), polyetherimide (PEI), or polypropylene glycol (PPG) having a molecular weight of 200 Da to 5,000 Da, or a polypeptide selected from the group consisting of polyglycine, polyhistidine, and poly(RADA) may be used. In non-limiting exemplary embodiment of the present disclosure, PEG of 1.0 kDA was used (Examples 1 and 2).

Another aspect of the present invention provides a hydrogel composition for controlling stem cell differentiation, the hydrogel composition including, as active ingredients, thermosensitive poly(phosphazene) to which a plurality of hydrophobic amino acids, hydrophilic polymers, and beta proteins such as Use of natural proteins generates problems related to stability and a financial pressure, and thus biomimetic peptides derived therefrom may be used. -cyclodextrin are substituted; and a stem cell differentiation regulator linked directly or via a linker to one or more molecules, as a guest molecule, selected from the group consisting of adamantine, azobenzene, cholesterol, tert-butyl, cyclohexyl ester, and naphthyl.

The terms used herein are the same as described above.

Further, the present invention provides use of the hydrogel composition in controlling stem cell differentiation, the hydrogel composition including, as active ingredients, thermosensitive poly(phosphazene) to which a plurality of hydrophobic amino acids, hydrophilic polymers, and beta proteins such as Use of natural proteins generates problems related to stability and a financial pressure, and thus biomimetic peptides derived therefrom may be used. -cyclodextrin are substituted; and a stem cell differentiation regulator linked directly or via a linker to one or more molecules, as a guest molecule, selected from the group consisting of adamantine, azobenzene, cholesterol, tert-butyl, cyclohexyl ester, and naphthyl.

As used herein, the term 'stem cells' collectively refers to undifferentiated cells at a stage prior to differentiation into cells constituting each tissue, and stem cells differentiate into specialized cells under specific differentiation stimulation (environment). Unlike differentiated cells of which cell division is stopped, stem cells have the characteristic of proliferation (expansion) because they are able to produce the same cells as themselves by cell division (self-renewal), and stem cells also have plasticity in differentiation, because they differentiate into specialized cells when differentiation stimulation is applied, and may also differentiate into different cells under different environment or different differentiation stimulation. These stem cells may be classified into embryonic stem cells and adult stem cells according to their origin, and in the present invention, adult stem cells are preferably used, rather than embryonic stem cells which have many biological, ethical, and legal problems and are limited in clinical applications. Among adult stem cells, mesenchymal stem cells (MSCs) rarely present in adult tissues such as bone marrow and adipose tissue may be used.

In the present disclosure, the use in controlling stem cell differentiation means a use in controlling stem cells to induce differentiation of stem cells to specialized cells such as chondrocytes, osteocytes, neurocytes, neuroblasts, muscle cells, adipocytes, etc.

The hydrogel composition for controlling stem cell differentiation of the present disclosure may be directly implanted to a disease site of an individual, together with stem cells, and may be cultured *in vitro.* For implantation, both a non-surgical treatment of using a catheter and a surgical treatment of injecting after incision of a disease site are possible.

As used herein, the 'stem cell differentiation regulator' refers to a chemical substance, a protein, or a peptide that influences stem cell differentiation. In particular, a material that exists in an extracellular matrix (ECM) and controls cell proliferation and differentiation may correspond thereto. Non-limiting examples thereof may include proteins such as BMP, N-cadherin, insulin-like growth factor (IGF), fibroblast growth factor (FGF), and transforming growth factor β (TGF-β). Use of natural proteins generates problems related to stability and a financial pressure, and thus biomimetic peptides derived therefrom may be used.

The stem cell differentiation regulator of the present disclosure may be a peptide including arginine-lysine-aspartic acid (RGD). In the hydrogel composition, when a ratio of the number of moles of RGD to the number of moles of the host molecule is in a range of 50% to 100%, the hydrogel composition may be a composition for inducing chondrocyte and/or osteocyte differentiation. Specifically, the hydrogel composition may induce differentiation in an early hypertrophic stage. When the ratio of the number of moles of RGD to the number of moles of the host molecule is in a range of 0% to 25%, the hydrogel composition may be a composition for inducing adipocyte differentiation.

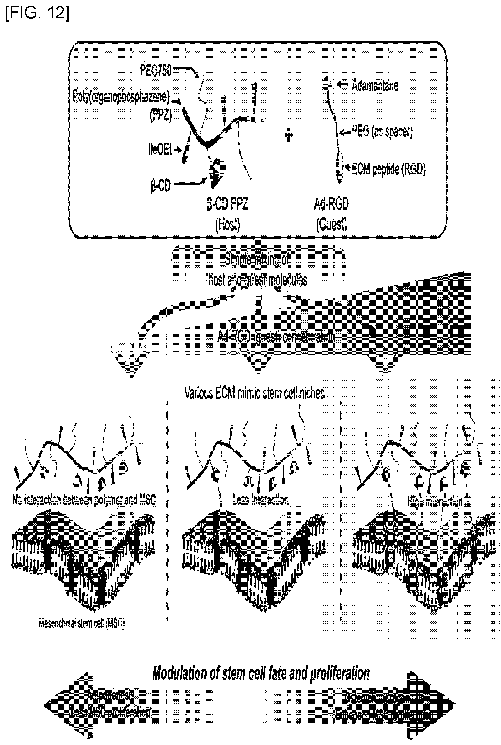

In this regard, in non-limiting exemplary embodiments of the present disclosure, among ECM molecule candidates, arginine-lysine-aspartic acid (RGD) which is an adhesive and developmentally effective peptide to MSC was selected as the stem cell differentiation regulator in the present invention (Example 1). RGD is a tri-peptide involved in MSC recognition, attachment, survival, and differentiation, and is a major binding site within fibronectin. Therefore, possessing of RGD in 3D hydrogel is necessary for survival and differentiation of MSC. RGD stimulation to MSC leads to various differentiation, such as osteocytes, chondrocytes, and adipocytes. According to Experimental Example 3 of the present disclosure, it was found that the fate of MSC may be controlled only by controlling the amount of Ad-RGD (see FIG. 12). This result suggests that it is necessary to design a highly controlled MSC niche, and RGD is needed as the physiologically active material, in order to provide a hydrogel composition for differentiation of MSCs into osteocytes, chondrocytes, and adipocytes.

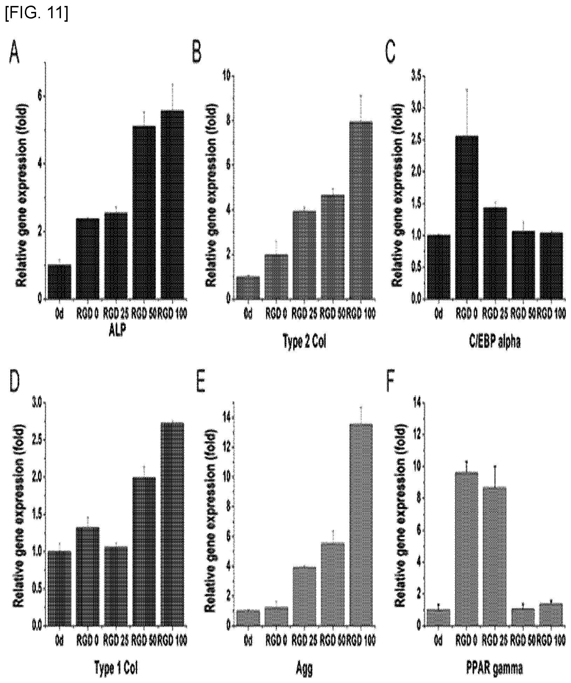

Further, in a situation where synthesis of hydrogel by controlling the concentration of various physiologically active materials present in ECM is required to attempt several synthesis batches, bone/cartilage/fat production could be induced by controlling the concentration of Ad-RGD as desired using host-guest interaction in the present disclosure (FIG. 11). This result suggests that the concentration of the physiologically active material to be finely controlled may be easily controlled in the hydrogel of the present disclosure.

Meanwhile, the stem cell differentiation regulator may be a peptide including CESPLKRQ and a peptide including CLRAHAVDIN. Here, the molar ratio of the peptide including CESPLKRQ and the peptide including CLRAHAVDIN may be in a range of 4:6 to 6:4. In this case, the hydrogel composition may be a composition for inducing differentiation into chondrocytes.

In this regard, TGF-β1 with a molecular weight of 25 kDa exists in the site of embryonic bone and cartilage development and has a critical role for the intracellular signaling cascade facilitating cartilage-specific gene expression. In particular, TGF-β1 regulates MAPK including p38, extracellular signal regulated kinase 1 (ERK 1), and c-Jun N-terminal kinase (JNK) as chondrogenesis controllers. In non-limiting exemplary embodiments of the present disclosure, CESPLKRQ was used, which is the most reactive binding site to TGF-β1 receptors in the whole peptide sequences of TGF-β1.

Next, a natural protein source for mimicking as a peptide is N-cadherin significant for cell to cell interaction and chondrogenesis. N-cadherin has a molecular weight of about 99.7 kDa. In the recent decade, the His-Ala-Val (HAV) motif inducing MSC chondrogenesis and mimicking N-cadherin action was highlighted in many studies. Although the sequence of HAV alone is enough to induce MSC chondrogenesis, an extended HAV sequence such as CLRAHAVDIN was shown to be more excellent as an effective motif. Hence, in non-limiting exemplary embodiments of the present disclosure, this extended HAV peptide sequence was chosen. Consequently, the present inventors chose a couple of peptide sequences derived from natural proteins such as TGF-β1 mimicking CESPLKRQ and N-cadherin mimicking CLRAHAVDIN under the stoichiometric peptide ratio control by a host-guest interaction. These peptides could affect MSCs to induce successive reaction of MSC with the mechanism of cell to cell interaction and intracellular signaling of mitogen activated protein kinase (MAPK), respectively.

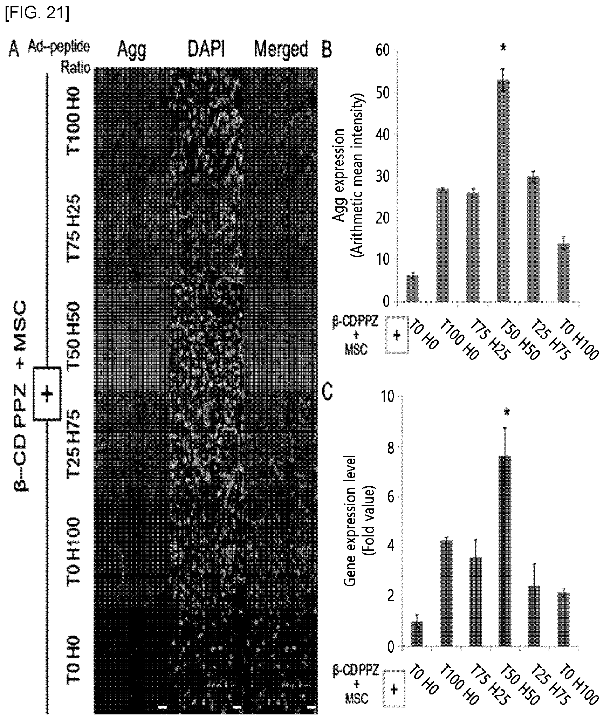

In Experimental Example 6 of the present disclosure, the highest chondrogenic gene and protein expression levels were observed in T50 and H50 (see FIGS. 20 and 21). In other words, synthesized adamantane-PEG-CESPLKRQ (Ad-TGF) and adamantane-PEG-CLRAHAVDIN (Ad-HAV) are used at the same time to induce optimized chondrogenesis. TGF-β1, orchestrating the elaborate control of MAPK factors (p38, ERK-1/2, and JNK), is quite related to the initiation of MSC chondrogenesis. N-cadherin also induces chondrogenesis of MSCs via the cell to cell interaction even in the synthetic hydrogel. MAPK activation for the chondrogenic differentiation may be harmonized by both TGF-β1 and N-cadherin. Since the chondrogenesis of MSC under the stimulation of TGF-β1 or N-cadherin alone had been elucidated, such proteins are crucial for chondrogenesis and correlated with each other for the intermediation of MAPK factors. The results of Experimental Example 6 mean that N-cadherin was not only allowing MSCs to condensate one other, but also influencing TGF-β1 to express chondrogenic genes such as Col II and Agg by MAPK. For these reasons, it could be concluded that both factors were not dominant over each other in terms of the development in chondrogenesis. Eventually, the harmony of these balanced two Ad-peptides such as T50 H50 group can induce an optimal chondrogenesis owing to their non-dominant characters for MAPK.

Still another aspect of the present invention provides a hydrogel composition for inhibiting cancer cell proliferation or metastasis, the hydrogel composition including, as active ingredients, thermosensitive poly(phosphazene) to which a plurality of hydrophobic amino acids, hydrophilic polymers, and beta-cyclodextrin are substituted; and IL-2 linked directly or via a linker to one or more molecules, as a guest molecule, selected from the group consisting of adamantine, azobenzene, cholesterol, tert-butyl, cyclohexyl ester, and naphthyl.

Additionally, still another aspect of the present invention provides use of a hydrogel composition in inhibiting cancer cell proliferation or metastasis, the hydrogel composition including, as active ingredients, thermosensitive poly(phosphazene) to which a plurality of hydrophobic amino acids, hydrophilic polymers, and beta-cyclodextrin are substituted; and IL-2 linked directly or via a linker to one or more molecules, as a guest molecule, selected from the group consisting of adamantine, azobenzene, cholesterol, tert-butyl, cyclohexyl ester, and naphthyl.

Meanwhile, the hydrogel inclusion complex of the present invention may be prepared by a process including a first step of preparing thermosensitive poly(phosphazene) to which a plurality of hydrophobic amino acids, hydrophilic polymers, and host molecules are substituted; a second step of preparing a physiologically active material linked to a guest molecule via a linker; and a third step of mixing the poly(phosphazene) with the physiologically active material.

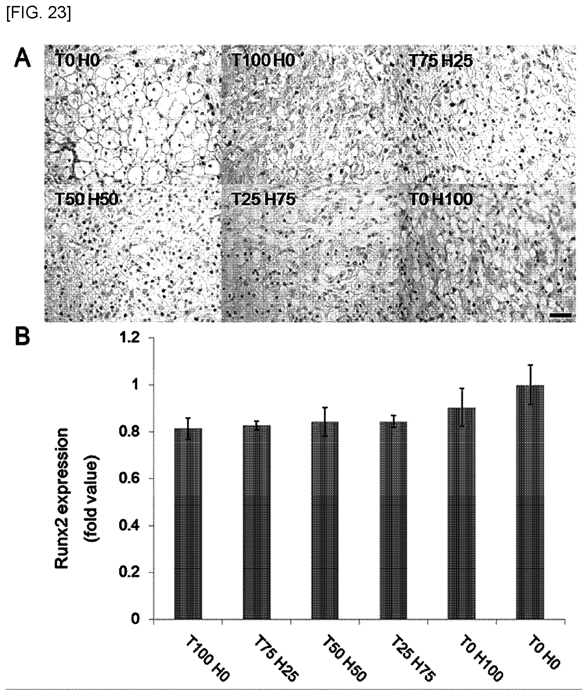

In the present disclosure, it was confirmed that the hydrogel composition for optimized chondrogenic differentiation of MSCs may be prepared without a further synthesis process after stoichiometrically controlling Ad-TGF and Ad-HAV, based on the host-guest interaction (FIG. 23).

Although some hydrogels were prepared with some Ad-peptide combinations for the illustrative purpose in the present disclosure, more Ad-peptide ratio combinations and sequences may also be used to easily prepare optimized niche for differentiation of stem cells to a desired state. Eventually, this technology provides a platform system by switching guest molecules or ratios to manufacture ideal 3D biomedical constructs. Accordingly, the hydrogel composition of the present disclosure, in which the kind, ratio, and sequences of the physiologically active material suitable for stem cell differentiation are controlled, may be easily prepared.

In this context, based on the composition of ECM known to be required for differentiation of stem cells to a specific state, known information about the kind and ratio of the physiologically active materials may be obtained, before the second step. Based on this information, the physiologically active material of the second step may be prepared according to the above information and ratio. Accordingly, the prepared hydrogel composition may be finely controlled such that it includes the physiologically active material according to the kind and ratio.

In the preparation method, when the number of moles of the physiologically active material is larger than the number of moles of the host molecule, a large amount of the physiologically active material is not bound, and thus the number of moles of the physiologically active material is preferably smaller than the number of moles of the host molecule.

Still another aspect of the present invention provides thermosensitive poly(phosphazene) to which a plurality of hydrophobic amino acids, hydrophilic polymers, and beta-cyclodextrin are substituted.

In this regard, the beta-cyclodextrin is characterized in that it is linked to the main chain of poly(phosphazene) via a hydroxyl group at C6 position of C₁₋₆ alkylene diamine, poly(C₁₋₆ alkylene diamine), n-amino-n-oxoalkanoic acid (wherein n is an integer of 2 to 6), thiol, carboxylate, C₂₋₆ hydroxyalkyl m-amino-m-oxoalkanoic acid (wherein m is an integer of 2 to 6), or cyano-amino-C₁₋₄ alkylthio-C₁₋₆ alkane as a linker. By including the beta-cyclodextrin linked via the linker of a suitable length having a predetermined functional group as described above, the influence on the gelling temperature of the thermosensitive poly(phosphazene) according to the substitution may be controlled to provide a thermosensitive gel which is gelled at a temperature near the body temperature.

Hereinafter, the present invention will be described in more detail with reference to the following Examples. However, these Examples are for illustrative purposes only, and the scope of the present invention is not intended to be limited thereby.

### Example 1: Hydrogel Composition Including β-CD PPZ and Adamantane-PEG-RGD

### Materials

Hexachlorocyclotriphosphazene (Aldrich) was purified by sublimation at 55°C under vacuum (about 0.1 mmHg). Poly(dichlorophosphazene) was prepared according to a known method (Sohn, Y. S. et al., Macromolecules 1995, 28 (22), 7566-7568). It was prepared from hexachlorocyclotriphosphazene using aluminum chloride (AlCl₃) as a catalyst at 250°C for 5 hr. L-Isoleucine ethyl ester hydrochloride (IleOEt·HCl) was prepared from L-isoleucine (Aldrich) according to a known method. α-Amino-ω-methoxy-poly(ethylene glycol) (AMPEG) with a molecular weight of 750 Da was prepared according to a known method (Loccufier, J.; Crommen et al., Die Makromolekulare Chemie, Rapid Communications 1991, 12 (3), 159-165). Tetrahydrofuran (THF) and triethylamine (TEA) (Junsei Chemical Co., Ltd.) were purified under the dry nitrogen atmosphere by refluxing at the boiling point over sodium metal/benzophenone (Acros) and barium oxide (Acros). β-Cydodextrin purchased from Aldrich was used without further purification. Mono-6-OTs-βCD and mono-6-diethylamino-βCD (NH₂-βCD) were synthesized according to a known method (Liu, Y.-Y.; Fan et al., Macromolecular Bioscience 2003, 3 (12), 715-719). Acetonitirile (ACN), ethanol amine (AEtOH), 4-(dimethylamino) pyridine (DMAP), isobutyl chloroformate (IBCF) and 1-ethyl-3-(3-dimethylaminopropyl)carbodiimide (EDC) were obtained from Aldrich. Dichloromethane (DCM) with an extra pure quality was purchased from Daejung chemical company (Korea) and used without further purification.

### (1) Synthesis of Acid PPZ

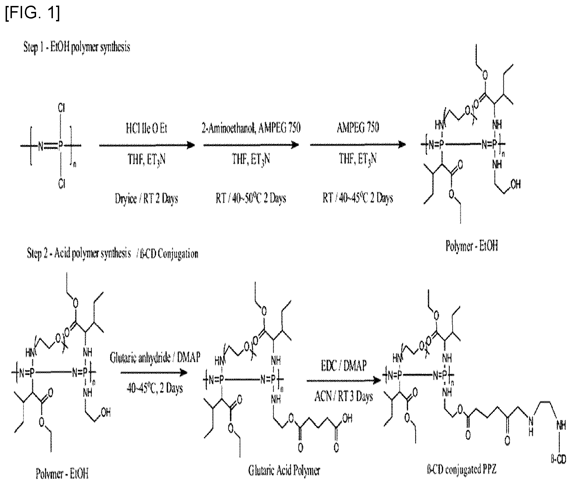

An acid polymer was synthesized as elucidated below. IleOEt·HCl (7.36 g, 0.038 mmol) suspended in dried THF (200 mL) containing TEA (16.54 mL, 0.12 mmol) was slowly added to poly(dichlorophosphazene) (3.00 g, 0.03 mmol) dissolved in dried THF (100 mL). The reaction mixture was stirred at the dry ice with acetone bath for 12 hr, and then kept stirring up to room temperature for 36 hr. To this mixture, AEtOH (2-aminoethanol, 0.46 ml, 0.008 mmol) dissolved in dried THF (20 mL) containing TEA (2.16 mL, 0.02 mmol), and AMPEG750 (7.57 g, 0.01 mmol) dissolved in dried THF (50 mL) containing TEA (4.92 g, 0.04 mmol) were gradually added, and the reaction mixture was stirred at room temperature for 24 hr, and then at 40°C to 50°C for 24 hr. The polymer reaction mixture was purified according to a known method. Briefly, the reaction mixture was filtered. After the filtrate was concentrated, it was poured into n-hexane to obtain a precipitate, which was re-precipitated in the same solvent system. The polymer product was dialyzed with a dialysis membrane (MW 12,000-14,000 cutoff) against methanol for 3 days, and distilled at 4°C for 4 days. The finally dialyzed solution was subsequently freeze-dried to obtain poly(organophosphazene) carrying AEtOH. Finally, the carboxylic acid-terminated polymer was obtained by following reactions. A solution of a polymer carrying AEtOH (5.84 g, 0.01 mmol) in distilled THF (200 mL) was stirred at room temperature under nitrogen atmosphere. To this polymer solution, each solution of glutaric anhydride (3.18 g, 0.02 mmol) and 4-(dimethylamino) pyridine (DMAP) (3.41 g, 0.02 mmol) in distilled THF (50 mL) was added. The reaction mixture was stirred at 40°C for 24 hr. After stirring, the reaction mixture was filtered and concentrated. The polymer product was purified by dialysis in methanol for 3 days and then by dialysis in distilled water at 4°C for 3 days. The dialyzed solution was freeze-dried to obtain poly(organophosphazene) carrying glutaric acid. A schematic illustration of the synthesis is shown in FIG. 1.

### (2) Synthesis of β-CD PPZ

An acid polymer (3.70 g, 6.77 mmol) was dissolved in ACN (100 mL) with stirring. Then, DMAP (2.48 g, 20.31 mmol), EDC (3.15 g, 20.31 mmol), and TEA (2.83 mL, 20.31 mmol) were added to the fully dissolved polymer solution. NH₂-βCD (0.54 g, 10.16 mmol) was dissolved in deionized water (10 mL). To activate the carboxyl group of the polymer, the deionized water solution of NH₂-βCD was added to the polymer solution 30 min after the addition of DMAP, EDC, and TEA. The reaction mixture was stirred at room temperature for 48 hr, and then evaporated under reduced pressure to collect the polymer. The collected polymer product was dissolved in methanol. The polymer product was dialyzed with a dialysis membrane (MW 12,000-14,000 cutoff) against methanol for 3 days and against deionized water at 4°C for 4 days, and the finally dialyzed solution was subsequently freeze-dried. A schematic illustration of the synthesis is shown in FIG. 1.

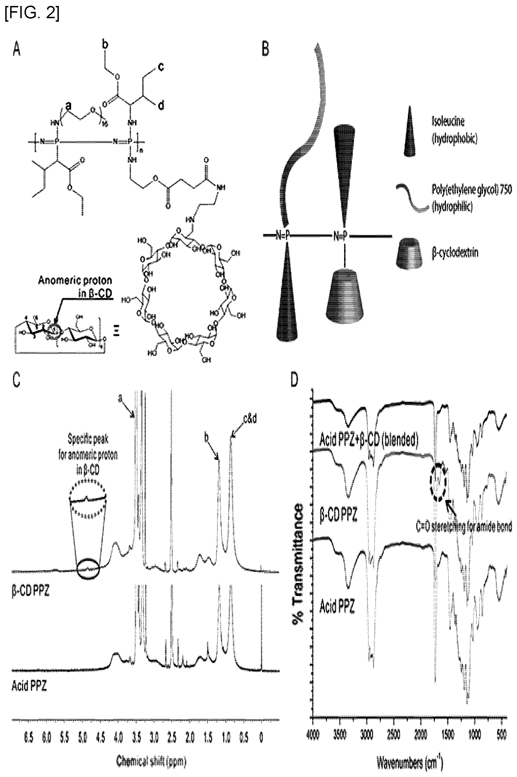

In the synthesized β-CD PPZ, the specific evidence for β-CD conjugation was observed with an anomeric proton peak of β-CD at 4.83 ppm after synthesis of β-CD PPZ (FIG. 2C). Furthermore, in FT-IR data, the unique peak resulted from β-CD conjugation was shown at 1,550 cm⁻¹ owing to a newly formed amide bond (C=O bond) between acid PPZ and β-CD (FIG. 2D). The synthesized β-CD PPZ has a distinctive feature of thermosensitivity, such as sol-gel transition, which can lead to molecular harmony between hydrophobic IleOEt and hydrophilic PEG, dependent on temperature. For example, hydrogen bonds of water in β-CD PPZ were broken with a temperature increment, and hydrophobic IleOEts then formed a strong hydrophobic interaction thereby forming a gel.

### (3) Synthesis of adamantane-PEG-MeAc

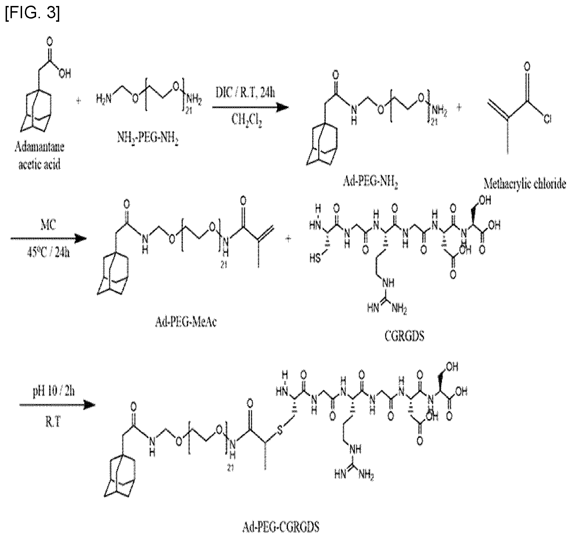

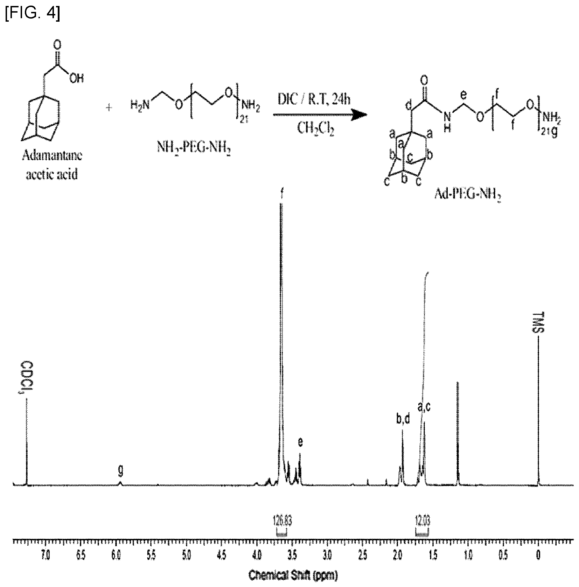

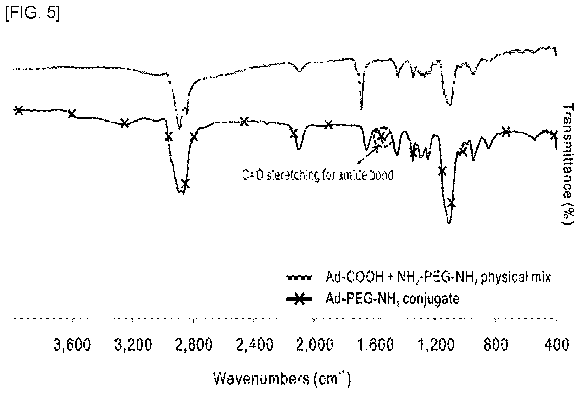

A schematic illustration of the synthesis is shown in FIG. 3. Adamantane acetic acid (1.07 g, 5.5 mmol) was dissolved in DCM. TEA (3.53 ml, 35 mmol) and DIC (2.35 mL, 18.6 mmol) were added to the solution. After stirring the solution at room temperature for about 30 min, NH₂-PEG 1K-N_{H2} (5.0 g, 5 mmol) was added and reacted at room temperature for 1 day. The solvent was removed under reduced pressure, and deionized water was added to precipitate the unreacted adamantine acetic acid. The precipitate was collected by centrifugation three times at 14,000 rpm for 20 min. Minuscule residual precipitate of adamantine acetic acid was filtered by a 0.45 µm paper filter. The unpurified white powder products were collected by lyophilization. Thin layer chromatography (TLC) was conducted to separate pure Ad-PEG-NH₂ from the unpurified white powder. The Rf value in TLC was 0.3 via the eluent composition (DCM:MeOH = 93:7). Based on the Rf value, the pure product of Ad-PEG-NH₂ dissolved in the eluent solvent was collected by liquid chromatography. The eluent solvent containing Ad-PEG-NH₂ was removed in an evaporation apparatus with reduced pressure. Ad-PEG-NH₂ was dissolved with distilled water, and then lyophilized to yield a white powder (3.19 g, 2.65 mmol). The product was analyzed by 400 MHz NMR (Bruker) and FT-IR. The results are shown in FIGS. 4 and 5.

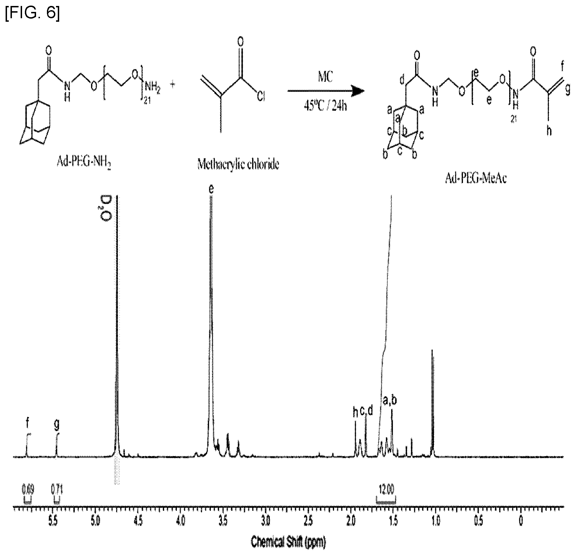

Ad-PEG-NH₂ (1 g, 0.8 mmol) was dissolved in DMC. Subsequently, methacryloyl chloride (0.97 mL, 9.3.mmol) and TEA (1.38 mL, 9.95 mmol) were added to the Ad-PEG-NH₂ solution. The reaction was performed at 50°C for 1 day. The resulting solution was purified by evaporation under reduced pressure, dialysis with MWCO 1 kDa, and lyophilization. The NMR value of the prepared Ad-PEG-MeAc is shown in FIG. 6.

### (4) Synthesis of adamantane-PEG-RGD (Ad-RGD)

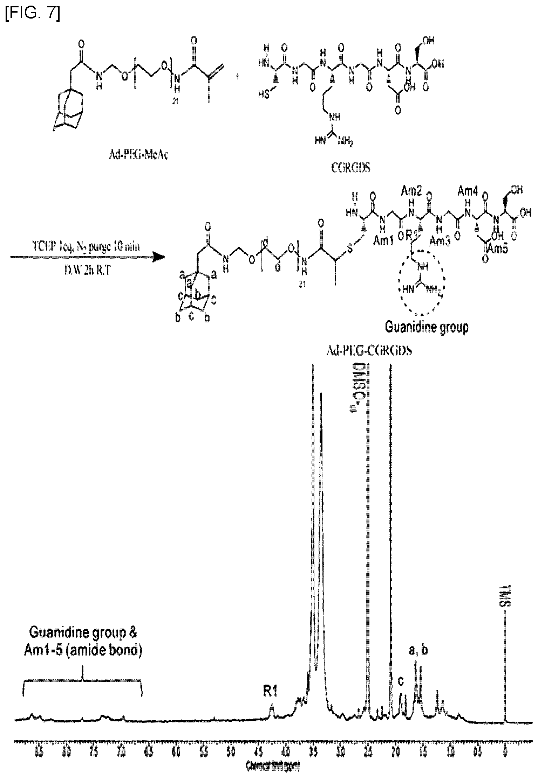

A schematic illustration of the synthesis is shown in FIG. 3. Ad-PEG-MeAc (0.3 g, 0.2 mmol) was dissolved in an aqueous solution of which pH was adjusted to 10.0. TCEP (66 mg, 0.23mmol) and CGRGDS (0.41 g, 0.69 mmol) were immediately added to the Ad-PEG-MeAc solution. The reaction solution was purged under N₂ atmosphere for 10 min. The reaction was performed for 2 hr at room temperature. Purification was performed with dialysis and lyophilization. The NMR value of the formed Ad-RGD is shown in FIG. 7.

### (5) Preparation of conjugate in which β-CD PPZ and Ad-RGD are mixed

Conjugates were prepared by mixing the prepared β-CD PPZ and Ad-RGD. The prepared conjugates included the Ad-RGD molecule at a concentration of 0%, 25%, 50%, or 100%, based on the number of host molecules present in the β-CD PPZ hydrogel. Ad-RGD 100, 50, and 25 mean that guest molecules are inserted to the host molecules of β-CD polymer through 100%, 50%, 25% host-guest interaction, respectively. Exact concentrations of Ad-RGD 100, 50, and 25 are described in Table 1 below.

**[Table 1]**

| Ad-RGD contents | Mole concentration (mole/mL) | Exact weight of Ad-RGD (mg) |
|---|---|---|
| Ad-RGD 100 | 6.88 × 10⁻⁶ | 12.9 |
| Ad-RGD 50 | 3.44 × 10⁻⁶ | 6.5 |
| Ad-RGD 25 | 1.72 × 10⁻⁶ | 3.2 |

As described, they were ultimately prepared in order to control the contents of stem cell adhesive moieties (i.e., RGD) in the 3D hydrogel space.

### (6) Characterization of β-CD PPZ / Ad-RGD

The structures of the prepared polymers were estimated by measuring ¹H NMR (Bruker avance III 400 MHz Fourier transform mode (DMSO-d₆ and CDCl₃). The viscosity of the aqueous polymer solutions was assessed on a Brookfield RVDV-III+ viscometer between 5°C and 70°C under a fixed shear rate of 0.1. The measurements were carried out with a set spindle speed of 0.2 rpm and with a heating rate of 0.33°C/min.

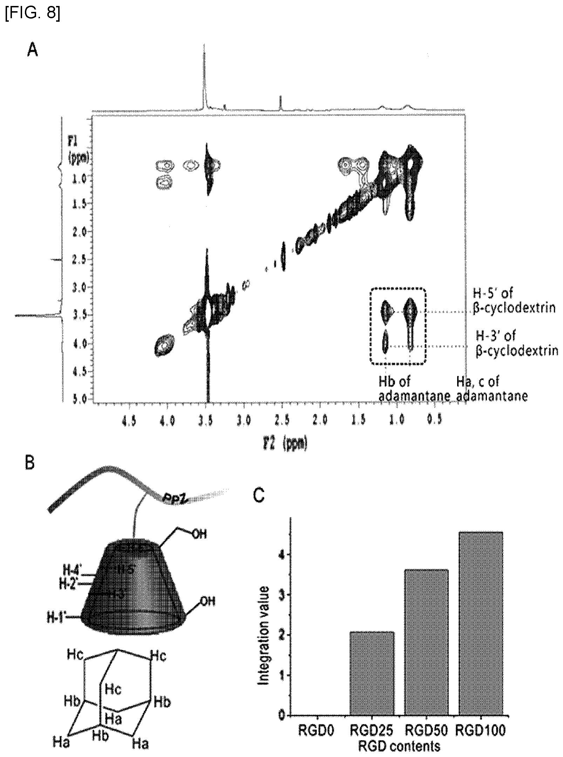

Spatial information was obtained from 2D-NMR (NOESY) with a 1:1 to 1:0 molar mixture of β-CD and adamantine containing RGD dissolved in DMSO-d₆. 2D-NMR spectra were recorded on a DD2 600 MHz FT NMR (Agilent Technologies), and are shown in FIG. 8.

### Experimental Example 1: Verification of Occurrence of Host-Guest Interaction by Control of Ad-RGD amount in Conjugate of Example 1

In this example, it was hypothesized that binding affinity between β-CD and Ad-RGD would induce the well-controlled guest molecule amount through the Ad-RGDs inserted at different amounts with one another. First of all, β-CD PPZ and β-CD PPZ + Ad-RGD gave different results from measurement of dynamic light scattering (DLS). The average particle size of β-CD PPZ and β-CD PPZ + Ad-RGD in an aqueous environment were 121.3 ± 26.2 nm and 180.3 ± 32.4 nm, respectively. As Ad-RGDs were included in β-CD PPZ, the practical size of aqueous particles of β-CD PPZ + Ad-RGD was increased.

To elucidate the direct evidence of the host-guest interaction, the integration values of 2D-NOESY of β-CD PPZ and Ad-RGD was measured with the increase of guest molecules. First of all, in the 2D-NOESY results, the inclusion complex was confirmed at cross peaks composed of Ad protons of methylene (Ha, Hc), methane (Hb), and β-CD inner cavity protons of H-5 (FIG. 8A). Consequently, the integration values of cross peaks between β-CD inner cavities and Ad was proportionally increased as the amount of guest molecules increased (FIG. 8C). From these results, it was confirmed that the host-guest interaction between β-CD PPZ and AD-RGD finely occurred, and thus, the occurrence of host-guest interaction can by controlled by stoichiometrically modulated Ad-RGD.

### Experimental Example 2: Thermosensitive Property of Conjugate of Example 1

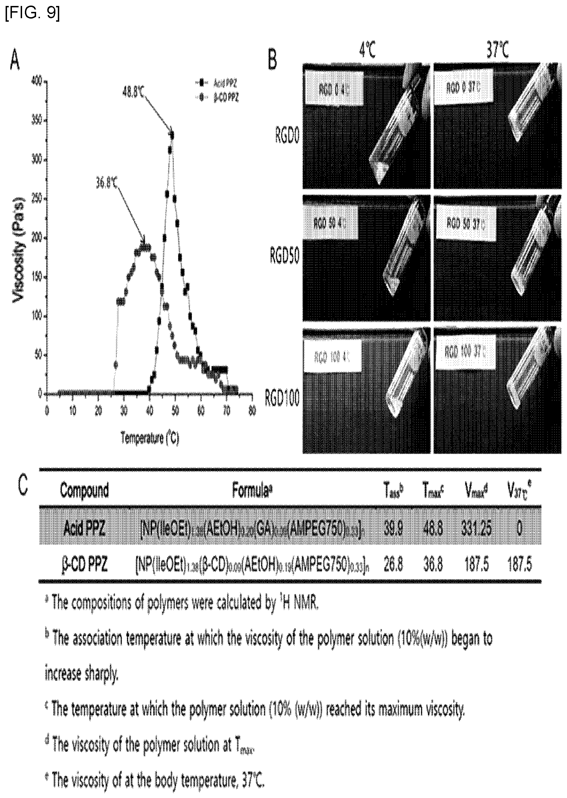

The viscosity change between acid PPZ and β-CD conjugate was observed. Acid PPZ Tₘₐₓ (temperature at which the solution reaches its maximum viscosity) was 48.8°C which is above the body temperature. The Tₘₐₓ of β-CD PPZ, in which hydrophilic carboxylic acid groups in acid PPZ were substituted with only β-CD possessing another hydrophilic moiety (i.e., OH group), was decreased to approximately 36.8°C (FIG. 9A).

Gelation after the preparation of the mixture of β-CD PPZ and Ad-RGD was examined. As a result, in a condition where the temperature is lower than the body temperature (4°C), the β-CD PPZ where Ad-RGD 0, Ad-RGD 50, and Ad-RGD 100 were added showed an aqueous state (FIG. 9B). When the temperature was increased to 37°C, well-formed gelation was observed in all groups. From these results, it was confirmed that Ad-RGDs, as guest molecule conjugates, do not affect gelation of β-CD PPZ hydrogel at the body temperature.

### Experimental Example 3: Measurement of Activity of Conjugate of Example 1 on Mesenchymal Stem Cell Viability and Differentiation Control

### (1) Cell culture method

Mouse mesenchymal stem cells (mMSCs) were purchased from Cyagen Biosciences Inc. mMSCs were cultured with Dulbecco's Modified Eagle's medium (DMEM) (Gibco BRL, Grand Island, NY) containing 1% penicillin-streptomycin (Sigma-aldrich, USA) and 10% fetal bovine serum (FBS) (Welgene, Korea) in dishes at 37°C in a humidified atmosphere of 5% CO₂ and 95% air.

### (2) Live/dead cell viability assay of mesenchymal stem cells & measurement of CCK-8 cultured with 3D hydrogel niche

Harvested mMSCs (passage 7, 5 × 10⁵ cells) were suspended in 0.1 mL of 10 wt% prepared hydrogel. mMSC/hydrogel mixtures were incubated in a 24-well culture plate with a cell insert.

At day 0, day 1, day 3, and day 7, the cell medium was removed, and calcein AM / ethidium homodimer-1 (Live/dead cell viability assay kit, Thermo Fisher Scientific Inc.) dissolved in a DPBS solution was used to perform a live/dead cell viability assay of mesenchymal stem cells. All images were obtained by a confocal microscope (Zeiss LSM 800, DE) in a 3D state.

For CCK-8 assay, on day 7, all of the 3D hydrogels including mMSCs were destroyed with media. The cultured hydrogel was moved to a 96-well culture plate (SPL life sciences, KR), and 10 µL of CCK-8 (Dojindo Molecular Technology, Inc. JP) solution was added to each well. CCK-8 solution containing the hydrogel was placed in a cell incubator in a humidified atmosphere at 37°C, 5% CO₂ for 2 hr. After incubation, the absorbance was measured using a microplate reader (BIO-RAD, Hercules, CA, USA) at a wavelength of 450 nm.

### (3) Gene assay of mesenchymal stem cells cultured with 3D hydrogel niche (RT-PCR)

An RNA extract was prepared by using Trizol (Invitrogen, Carlsbad, CA). After samples were treated with DNase (Invitrogen), 1 mg of total RNA was used for cDNA synthesis (Superscript First-strand synthesis system, GibcoBRL, Life Technologies). In brief, a reverse transcription reaction was carried out in a 20 mL mixture (1 RT buffer, 1.25 mM MgCI2, 5 mM DTT, 2.5 g random hexamer, 0.5 mM each of dATP, dCTP, dGTP, and dTTP, and 50 U of Superscript II enzyme). After the reverse transcription reaction, RNA was degraded by 2 U of *Escherichia* coli RNase H. PCR was performed in 50 mL of a reaction buffer containing 2 U of Takara Taq, 1× PCR buffer, 0.8 mM dNTP mixture, and 100 pmol of specific primers. Standard PCR conditions were as follows: at 95°C for 3 min, followed by cycles of denaturation at 95°C for 5 sec, annealing at 60°C for 34 sec, and extension at 72°C for 1 min. Oligonucleotides used as primers are described in Table 2. The gene expression values were normalized against the housekeeping gene of β-actin.

**[Table 2]**

| Name | Forward primer | Reverse primer |
|---|---|---|
| **ALP** | CGC CAG AGT ACG CTC CCG CC | TGT ACC CTG AGA TTC GT |
| **Collagen I** | GAA GTC AGC TGC ATA CAC | AGG AAG TCC AGG CTG TCC |
| **Collagen II** | GCG GTG AGC CAT GAT CCG CC | GCG ACT TAC GGG CAT CCT |
| **Aggrecan** | GAA ATG ACA ACC CCA AGC AC | TCT CCG CTG ATT TCA GTC CT |
| **C/EBPα** | GGA ACT TGA AGC ACA ATC GAT C | TGG TTT AGC ATA GAC GTG CAC A |
| **PPARγ** | GCT GTT ATG GGT GAA ACT CTG | ATA AGG TGG AGA TGC AGG TTC |

### (4) Experimental Results

A simple mixing mediated RGD concentration controllable system was designed to manufacture a stem cell 3D niche based on a host-guest interaction. The main integrin-binding domain, RGD, is abundant in ECM proteins such as fibronectin, vitronectin, fibrinogen, osteoponin, and bone sialoprotein. The manufactured 3D hydrogel stem cell niche was regulated by the amount of guest molecule, Ad-RGD. MSC survival rate was evaluated in recent studies with 3D scaffold of several synthetic materials, and the results showed 50% or higher viability within specific conditions.

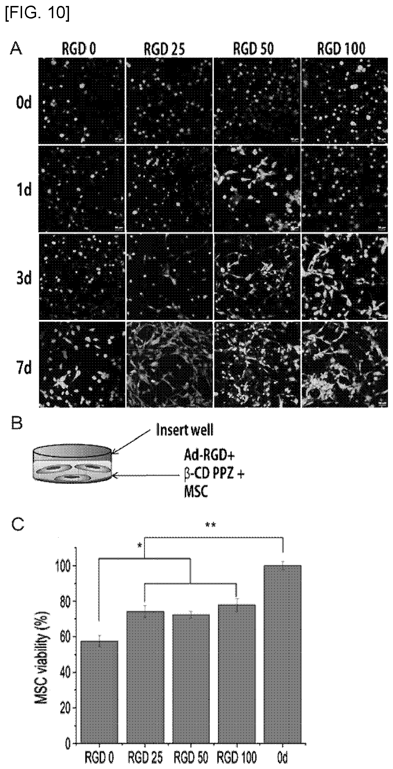

The results of evaluating the survival rates of MSCs in the stem cell culture system are shown in FIG. 10C. In live/dead cell viability assay images, MSCs encompassed with host and guest molecules were fairly alive in the 3D culture system for 7 days. In addition, the grown morphology of MSCs was observed in the presence of RGD (specifically, in Ad-RGD 25, 50, and 100 groups) owing to the RGD-integrin binding (FIG. 10A). MSC survival rates at 7 days after 3D culture were also more elevated in the presence of RGD 25, 50, and 100 (survival rate: 72.2% to 77.8%), compared to RGD 0 (survival rate: 57.5%). However, MSC survival rates among Ad-RGD 25, 50, and 100 were similar to each other. From these results, it was confirmed that the MSC survival rate is affected by the presence/absence of RGD. In other words, this result suggests that the presence of RGD molecule plays an important role in stem cell survival even in the synthetic 3D stem cell niche.

Conjugates in which Ad-RGDs of 0%, 25%, 50%, and 100% based on the number of moles of β-CD in the β-CD PPZ hydrogel were added were fabricated according to Example 1, and the conjugates were added to MSCs.

With regard to MSC differentiation, when Ad-RGD 50 and 100 were used, high binding possibility between Ad-RGD and α₅β₁ integrin of MSCs resulted in enhanced gene expression of osteogenic factors (ALP and collagen I) and chondrogenic factors (aggrecan and collagen II) (FIGS. 11A, 11B, 11D, and 11E). During development, chondrogenesis process temporarily precedes osteogenesis, and then hypertrophic stage is an intermediate gateway between these processes. In detail, spatiotemporal chondrogenesis-related factors and osteogenesis-related factors are simultaneously enhanced in the hypertrophic stage. In other words, when RGD 50 and RGD 100 were used, osteogenic factors were greatly increased, and when RGD 100 was used, chondrogenic factors were significantly increased, and it was confirmed that MSCs cultured with Ad-RGD 50 and 100 were confronted with the hypertrophic stage. Especially, chondrogenic factors were shown highin Ad-RGD 100, and it was confirmed that the high level of RGD was resulted in early hypertrophic stage, compared to the use of relatively low Ad-RGD 50.

On the other hand, adipogenesis-related factors (C/EBPα and PPARγ) were expressed highly in the low level or in the absence of Ad-RGDs such as RGD 0 and RGD 25 (FIGS. 11C and 11F). Consequently, the fate of MSCs can be controlled by only by the control of the amount of Ad-RGD bound to β-CD PPZ.

From these results, a schematic illustration of MSC differentiation activity of the conjugate of β-CD PPZ and Ad-RGD of the present invention is shown in FIG. 12.

### Example 2: Hydrogel Composition Including β-CD PPZ, Ad-TGF, and Ad-HAV

### (1) Synthesis of β-CD PPZ

β-CD PPZ was prepared in the same manner as described in (1) and (2) of Example 1.

### (2) Synthesis of Ad-PEG-MeAc

Ad-PEG-MeAc was prepared in the same manner as described in (3) of Example 1.

### (3) Synthesis of Ad-TGF

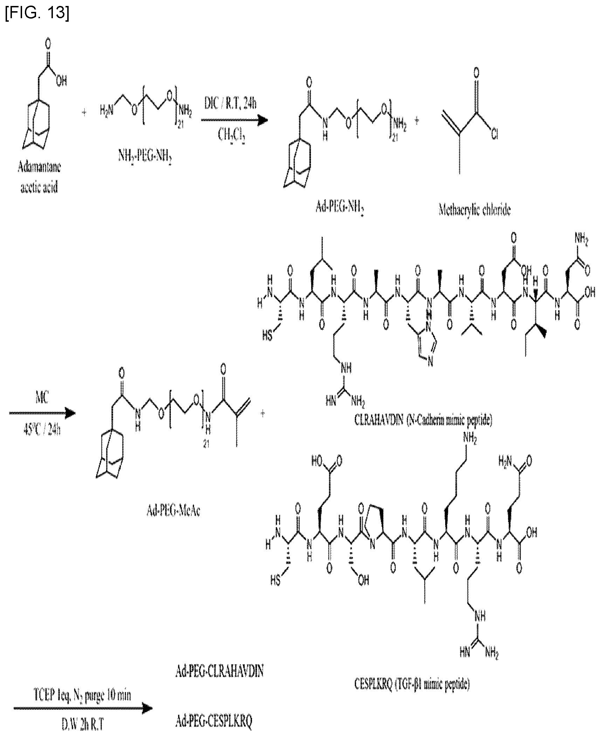

The prepared Ad-PEG-MeAC (M.W. 1302.6 Da, 200 mg, 0.15 mmol) was dissolved in an aqueous solution of which pH was adjusted to 10.0. Tris(2-carboxyethyl)phosphine (TCEP) (44.0 mg, 0.15 mmol) and TGF-β1 mimic peptide (CESPLKRQ, 960.12 Da, 442.2 mg, 0.46 mmol) were simultaneously added to the aqueous solution. The reaction solution was purged in N₂ atmosphere for 10 min. The reaction was performed for 2 hr at room temperature. After reaction, purification was carried out with dialysis and lyophilization. A schematic illustration of the synthesis is shown in FIG. 13.

### (4) Synthesis of Ad-HAV

The prepared Ad-PEG-MeAc (M.W. 1302.6 Da, 300 mg, 0.23 mmol) was dissolved in an aqueous solution of which pH was adjusted to 10.0. TCEP (66.0 mg, 0.23 mmol) and N-cadherin mimic peptide (CLRA**HAV**DIN, 1,111.29 da, 767.8 mg) were simultaneously added to the aqueous solution. The reaction solution was purged in N₂ atmosphere for 10 min. The reaction was performed for 2 hr at room temperature. After reaction, purification was carried out with dialysis and lyophilization. A schematic illustration of the synthesis is shown in FIG. 13.

### (5) Preparation of conjugates by mixing β-CD PPZ with Ad-TGF and Ad-HAV

The prepared β-CD PPZ was mixed with Ad-TGF and Ad-HAV in the ratio of the following Table 3 to prepare conjugates.

**[Table 3]**

| Abbreviation | Amount of molecules actually consumed (mg)* | | Abbreviation | Amount of molecules actually consumed (mg)* | |
|---|---|---|---|---|---|
| | TGF | HAV | | TGF | HAV |
| T100 H0 | 1.10 | 0 | T25 H75 | 0.28 | 0.90 |
| T75 H25 | 0.83 | 0.30 | T0 H100 | 0 | 1.20 |
| T50 H50 | 0.55 | 0.60 | T0 H0 | 0 | 0 |

| | | | | | |
|---|---|---|---|---|---|
| *Amount of Ad peptides actually consumed per 0.01 g of β-CD PPZ | | | | | |

### (6) Characterization of β-CD PPZ / Ad-TGF and(or) HAV

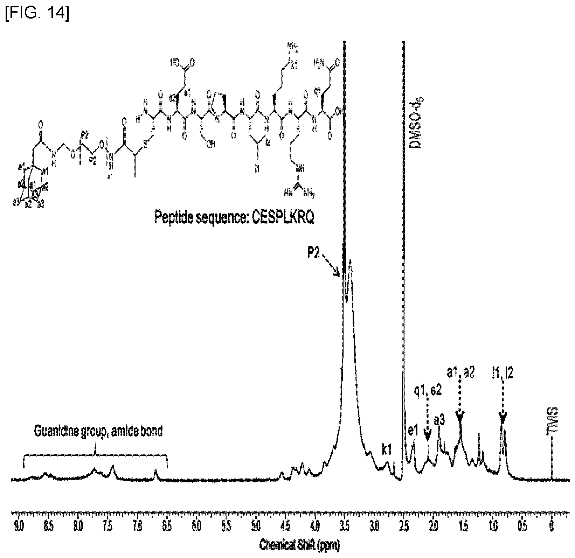

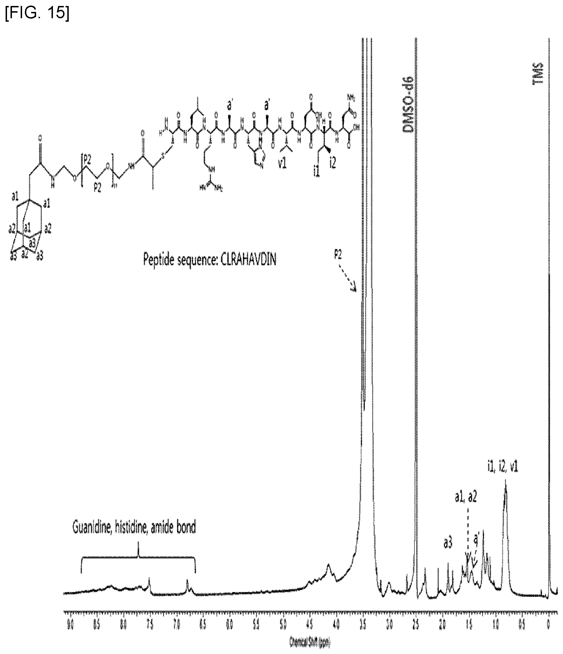

The structures of the prepared β-CD PPZ, Ad-HAV, and Ad-TGF were estimated by measuring ¹H NMR (Bruker avance III 400 MHz Fourier transform mode with DMSO-d₆ and CDCl₃) (FIGS. 2, 14, and 15).

The viscosity of the aqueous polymer solutions was assessed on a Brookfield RVDV-III+ viscometer between 5°C and 70°C at a fixed shear rate of 0.1. The measurements were carried out with a set spindle speed of 0.2 rpm and with a heating rate of 0.33°C/min (FIG. 16A).

Spatial information was obtained from 2D-NMR (NOESY) with a 1:1 molar mixture of β-CD and adamantine containing peptides dissolved in D₂O. A2D-NMR spectra were recorded on DD2 600MHz FT NMR (Agilent Technologies) (FIG. 16C).

### Experimental Example 4: Verification of Host-Guest Interaction Occurrence between β-CD PPZ and Ad-peptides in Conjugates of Example 2

To verify the host-guest interaction between β-CD PPZ and Ad-peptides, 2D NOESY spectra in an aqueous state with D₂O under the same conditions of the prepared conjugate hydrogels were measured. 2D NMR is the strongest method of observing the intermolecular interactions and/or the configuration of an inclusion complex. Cross peaks in 2D-NOESY could be obtained from nuclei resonance connections that are spatially closer than a coupled bond. Ha, c, and Hb (δ 0.6 ppm to 1.3 ppm) of adamantane and H-5' (δ 3.4 ppm to 3.5 ppm) of β-CD inner cavity, which are cross peaks in 2D-NOESY spectra involved in host-guest interactions, were fairly elucidated in FIG. 16C.

Further, to identify whether Ad-peptides were stoichiometrically and accurately inserted into the host molecules, dynamic light scattering (DLS) measurement was performed by mixing host molecules and guest molecules such that the ratio of host molecule : guest molecule was 1:1, 1:1.2, and 0:1 . Above all, the increased hydrodynamic diameters were observed in the conjugates in which the ratio of host molecule : guest molecule was 1:1 (FIG. 17C). In this DLS result of the conjugate in which the ratio of host molecule: guest molecule was 1:1, any peaks involved in guest molecules alone were not shown (FIG. 17C). It means that there were no guest molecules separated from β-CD PPZ. Since particle sizes of Ad-TGF and Ad-HAV alone were 353.63 ± 31.71 nm and 460.78 ± 49.53 nm, respectively (FIG. 17E), and these much larger particle sizes of Ad-TGF and Ad-HAV, compared to β-CD PPZ, were resulted in the aggregation of hydrophobic adamantane molecules in an aqueous state. This means that all of the Ad-peptides were incorporated into the conjugates of β-CD PPZ and Ad-peptide (host molecule: guest molecule= 1:1) in the aqueous environment. On the other hand, it was confirmed that when Ad-peptides was excessively mixed with β-CD PPZ (host molecule: guest molecule = 1:1.2), the surplus Ad-peptides formed their own other peaks analogous to those of Ad-TGF and Ad-HAV alone (FIG. 18). Consequentially, it was confirmed from the 2D NOESY result that the conjugates were well formed due to the host-guest interaction between β-CD PPZ and Ad-peptide.

Meanwhile, whether the conjugates in which Ad-TGF and Ad-HAV were initially bound at different contents in β-CD PPZ could be maintained was examined using an *in vivo* imaging system (IVIS) with live animals. In the molecular tails of Ad-TGF and Ad-HAV, rhodamine (Rho) and fluorescein isothiocyanate (FITC) were linked in a conjugation process, respectively. Then, these fluorescence expressing guest molecules were injected along with β-CD PPZ in MSCs post-inclusion complex fabrication. Since β-CD in PPZ was released to the outside owing to biodegradability or dissolution of PPZ, and the Ad-peptide signals containing fluorescence became smaller over time. Furthermore, the stoichiometric control patterns of Ad-TGF/Ad-HAV were observed to employ different guest molecules for 21 days (FIG. 19A). For instance, fluorescence intensities of Ad-TGF were high in this order of T100 H0, T75 H25, T50 H50, and T25 H75. This result was derived from stoichiometrically different guest molecules holding dependent on the host-guest interaction. Then, the region of interest (ROI) value for the expressed fluorescence was calculated as shown in FIG. 19B. As Ad-peptides were mixed with the acidic PPZ (lack of β-CD in PPZ), the fast escape of Ad-peptides from 3D hydrogels occurred. Although, in all of the groups (T100 H0, T75 H25, T50 H50, T25 H75, and T0 H100), ROI values are decreased over time due to biodegradability and dissolution of β-CD PPZ, but the controlled Ad-peptide fluorescence expression in β-CD PPZ was maintained by the ratio of Ad-peptides, which were initially added differently, among all of the groups and all of the time points due to the host guest interaction. This result indicates considerably long-term maintenance of host-guest interaction in 3D β-CD PPZ hydrogel even in live animals.

### Experimental Example 5: Thermosensitive Property

Following the synthesis of host and guest molecules, the rheological measurement and the visualization of thermosensitive sol gel transition with β-CD PPZ incorporating T100 H0 and T0 H100 were performed. Even when Ad-TGF 100 and Ad-HAV 100 were incorporated into β-CD PPZ, hydrogels of the prepared conjugates also showed the gelation properties and enough viscosity in the body temperature (FIG. 16). T values of β-CD PPZ / Ad-TGF 100, and β-CD PPZ / Ad-HAV 100 prepared via the host-guest interaction were shifted to a slightly higher temperature, compared to the β-CD PPZ itself, owing to possession of hydrophilic PEGs in Ad-TGF and Ad-HAV (FIG. 16A). In particular, β-CD PPZ, β-CD PPZ / Ad-TGF 100, and β-CD PPZ / Ad-HAV 100 showed viscosity values of 268.75, 387.50, and 325.00 pa.s at the body temperature, respectively (FIG. 16A). Based on this thermosensitive viscosity result, the hydrogels prepared in Example 2 can be appropriately injected to live animals.

### Experimental Example 6: Histocompatibility of Conjugate of Example 2 and Chondrogenesis Induction of MSCs

### (1) Measurement method of biocompatibility

mMSCs (mouse mesenchymal stem cells) and NIH3T3 (mouse fibroblast cells) with a constant density (1 × 10⁴ cells/well) were seeded in a 96-well tissue culture plate (SPL, Korea). Each cell line was incubated for 24 hr with the well dissolved β-CD PPZ (concentration: 0 µg/mL to20,000 µg/mL). After incubation, the medium used was discarded and cells were washed once with DMEM and fresh PBS. After adding a fresh medium (200 mL/well), 3-(4,5-dimethylthiazol-2-yl)-2,5 diphenyltetrazoliumbromide (MTT) solution (100 mg/well) was added to the cells followed by incubation for 4 hr at 37°C in a humidified atmosphere of 5% CO₂. The formed formazan crystals were solubilized by incubating the cells with DMSO. The absorbance of the solution was measured at 570 nm using a microplate reader (Bio-Tek Instruments, USA). The cell viability (%) was calculated from [ab]test/[ab]control × 100%.

### (2) In vivo tissue generation test

All of the experiments with live mice were accomplished in compliance with the relevant laws and institutional guidelines of Institutional Animal Care and Use Committee (IACUC) in Korea Institute of Science and Technology (KIST). IACUC approved the experiment (approval number: 2017-092). Balb/c nude mice (4 weeks old, 20-25 g, female) were purchased from Nara Bio INC. (Gyeonggi-do, Korea). Nude mice were anaesthetized with 3% isoflurane in the balanced oxygen and nitrogen. MSCs/ β-CD PPZ (10 wt%)/ 0% to 100% of Ad-TGF (T, adamantane-PEG1000-CESPLKRQ, 2,248.70 Da) and Ad-HAV (H, adamantane-PEG1000-CLRAHAVDIN, 2,399.87 Da) were injected into subcutaneous pockets in mice on their right sides lateral to the dorsal midline using a syringe with a 31 gauge needle. Each mouse received a 100 µL injection containing 2 × 10⁶ cells mixed with 10 wt% of β-CD PPZ. All the tissues were collected 4 weeks post injection, and were used for histological examinations and gene analyses.

### (3) Histological and immunohistological analyses

All the collected tissues were embedded in paraffin, and sectioned with a microtome (6 µm in thickness). For histological evaluation, tissue sections were deparaffinized, rehydrated, and stained with H&E, safranin-O, Von kossa, and immunohistochemistry. For immunohistochemical staining, the sectioned tissues were incubated at 4°C overnight with primary antibodies of anti-aggrecan (1:500, Abcam, ab3778) and anti-collagen II (1:500, Abcam, ab34712). After washing three times, the slides were incubated with appropriate secondary antibodies conjugated to fluorescent dyes, such as goat anti-mouse IgG (TRICT, Abcam, ab6786) and goat anti-rabbit IgG (Alexa 488, Abcam, ab150077). Images were captured using a confocal laser scanning microscope (Zeiss) and a bright imaging microscope (Zeiss).

### (4) Gene assay measured in artificial tissue

An RNA extract was prepared by using Trizol (Invitrogen, Carlsbad, CA). After tissues were treated with DNase (Invitrogen), 1 mg of RNA was used for cDNA synthesis (Superscript First-strand synthesis system, GibcoBRL, Life Technologies). In brief, a reverse transcription reaction was carried out in a 20 mL mixture (1 RT buffer, 1.25 mM MgCl₂, 5 mM DTT, 2.5 g of random hexamer, 0.5 mM each of dATP, dCTP, dGTP, and dTTP, and 50 U of Superscript II enzyme) at 42°C. After the reverse transcription reaction, RNA was degraded by 2 U of *Escherichia coli* RNase H. PCR was performed in a 50 mL reaction buffer containing 2U of Takara Taq, 1 × PCR buffer, 0.8 mM dNTP mixture, and specific primers of 100 pmol. Standard PCR conditions were as follows: at 95°C for 3 min, followed by cycles of denaturation at 95°C for 5 sec, annealing at 60°C for 34 sec, and extension at 72°C for 1 min. Oligonucleotides used as primers are as described in Table 4 below. The gene expression values were normalized against the housekeeping gene of β actin.

**[Table 4]**

| Name | Forward primer | Reverse primer |
|---|---|---|
| **Collagen II** | GCG GTG AGC CAT GAT CCG CC | GCG ACT TAC GGG CAT CCT |
| **Aggrecan** | GAA ATG ACA ACC CCA AGC AC | TCT CCG CTG ATT TCA GTC CT |
| **Runx2** | GCG TCA ACA CCA TCA TTC TG | CAG ACC AGC AGC ACT CCA TC |
| **β-actin actin** | ACT CTT CCA GCC TTC CTT CC | ACT CGT CAT ACT CCT GCT TGC |

### (5) Experimental results

According to (1) described above, an *in vitro* cytotoxicity test was performed using MTT assay in both cell lines of MSCs and NIH3T3. Although each cell line was treated with a high concentration (10,000 µg/mL) of a β-CD PPZ polymer solution, the cell viabilities of MSCs and NIH3T3 still remained high to be above 80%. Therefore, this polymer was regarded as appropriate for the next study of *in-vivo* animal test.

In the *in-vivo* tissue generation test, the biocompatibility in the body administered with β-CD PPZ/ratio-controlled Ad-peptides/MSCs was confirmed using H&E staining. According to the histological results from H&E staining, any foreign body response such as a generation of foreign body giant cells and any toxicity were not observed in all of the groups treated with β-CD PPZ/ratio-controlled Ad-peptides (FIG. 20A). Then, neo-chondrogenesis of ectopically injected β-CD PPZ/ratio-controlled Ad-peptides/MSCs was observed using safranin-O staining. Cartilages and cytoplasm were stained red and green, respectively. The red color stained in cartilage was quite distinctly observed in all of the groups, except the T0 H0 group (FIG. 20A).

Moreover, MSC maintenance in living bodies were measured to examine whether local MSC maintenance enhances their therapeutic efficiency. Hence, MSCs capable of expressing green fluorescent protein (GFP) were encompassed with the inclusion of β-CD PPZ/Ad-peptide conjugate to confirm whether these MSCs are maintained in living animals. Locally injected MSCs were maintained in their injection sites for a period of 21 days. However, the significantly low maintenance of MSC fluorescence was monitored in the T0 H0 group in which both a receptor mediated MSC attachment and a cell to cell interaction available factor were excluded (FIG. 20B), suggesting that cell to cell interaction and TGF-β1 stimulation can enhance the survival rate of stem cells as well as chondrogenic differentiation. Evaluation of the region of interest (ROI) value for the remaining MSCs was also confirmed this low MSC maintenance in the T0 H0 group, compared to other Ad-peptide containing groups (FIG. 20C). The presence of Ad-peptides with MSCs assisted the local MSC maintenance in injected sites. In brief, the stoichiometrically incorporated β-CD PPZ hydrogels of these guest molecules were biocompatible in both *in vitro* and *in vivo* levels. Moreover, MSC chondrogenesis inductions using the β-CD PPZ/Ad-peptide complex was also observed in groups other than the T0 H0 group.

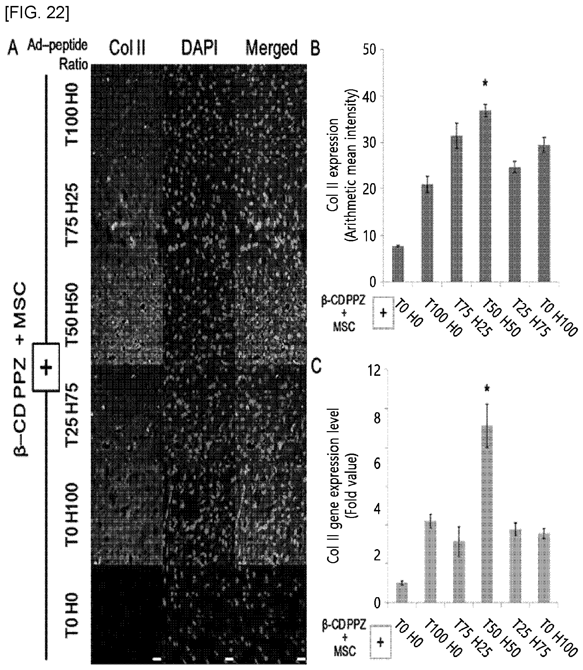

To evaluate exact MSC chondrogenesis levels in the flexible and different guest molecule contents, an analysis was performed with the typical chondrogenesis markers of type II collagen (Col II) and aggrecan (Agg). Based on the output, MSC chondrogenic differentiation with flexibility of guest molecules was shown in results of safranin-O staining. All of the groups, except the T0 H0 group, showed chondrogenic differentiation. Based on the results of safranin-O staining, the accurate chondrogenesis consequences were evaluated using the measurement of gene expression levels and immunohistochemistry. Neotissues resulting from the locally and subcutaneously injection of MSCs encompassed by β-CD PPZ and Ad-peptides were extracted from all of the experiment groups. Agg which is a cartilage-specific proteoglycan core protein was significantly synthesized and expressed in the all Ad-TGF and/or Ad-HAV incorporating groups. In particular, the highest Agg-specific fluorescence and gene expression levels were observed in T50 H50, compared to T100 H0, T75 H25, T25 H75, and T0 H100 groups (FIGS. 21B and 21C). The gene and protein expression levels in T50 and H50 were almost twice as high, compared to other gene groups, such as T100 H0, T75 H25, and T25 H75. In the absence of Ad-peptide, there was no Agg expression. Col II, which is a major protein for cartilage, was selected as the next marker for the chondrogenesis evaluation. Col II was also evaluated in terms of specific fluorescence and gene expressions. Col II was well formed in the β-CD PPZ/Ad-peptide-treated group, compared to T0 H0 group (FIG. 22A). Col II gene and protein expression showed a similar pattern with Agg in measurements of immunohistochemistry and gene assay (FIGS. 22B and 22C). Furthermore, the highest expression of Col II was also shown in the T50 H50 group.

Osteogenesis is a single continuous development process including cartilage formation as a precursor. In detail, even if hypertrophic chondrocytes are differentiated to osteocytes under specific stimulations such as runt-related transcription factor 2 (Runx2) and osterix, Ad-peptides used in the present disclosure should prevent any further process of chondrocytes to osteogenic termination. Inhibition of osteogenesis is important because the incorrect differentiation product at the terminal MSCs during the chondrogenesis process. Generally, Runx2 is considered as a typical and key transcription factor for osteogenesis. Hence, Runx2 was selected as a gene assay marker for detecting a further progress of osteogenesis. Furthermore, production of calcium, which is a a final product of osteogenesis, was confirmded via Von kossa staining. As a result, there was no calcium dot (FIG. 23A). Furthermore, no excessive level of Runx2 gene expression was detected (FIG. 23B). Eventually, osteogenesis in the system of Example 2 was perfectly blocked even using a TGF-β1 derived peptide, and chondrogenesis induction was observed only in groups where Ad-peptides were incorporated.

From these results, a schematic illustration of MSC differentiation activity of β-CD PPZ/ratio-controlled Ad-TGF/Ad-HAV conjugate of the present invention is shown in FIG. 12.

### Example 3: Conjugate of β-CD PPZ and Ad-IL2 via Host-Guest Interaction

### (1) Synthesis of host molecule, β-CD PPZ

β-CD PPZ was prepared in the same manner as described in (1) and (2) of Example 1.

### (2) Synthesis of Ad-PEG-MeAc

Ad-PEG-MeAc was prepared in the same manner as described in (3) of Example 1.

### (3) Synthesis of Ad-IL2

### 1) Method 1 (via click chemistry)

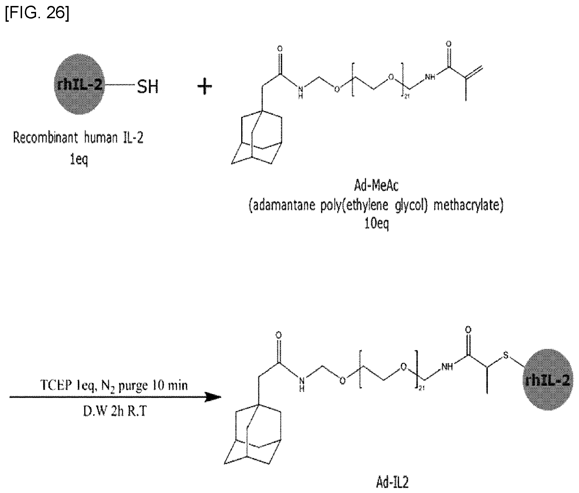

The prepared Ad-PEG-MeAc (0.09 mg, 0.00007 mmol) was dissolved in an aqueous solution of which pH was adjusted to 10.0. TCEP (0.2 mg, 0.0007 mmol) and rhlL-2 (1 mg) were added simultaneously to the aqueous solution. The reaction solution was purged in N₂ atmosphere for 10 min. The reaction was performed for 2 hr at room temperature. After reaction, purification was carried out with dialysis and lyophilization. A schematic illustration of the synthesis is shown in FIG. 26.

### 2) Method 2 (via EDC chemistry)

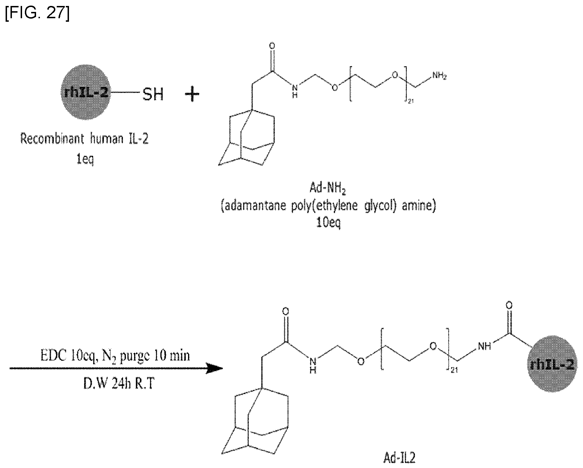

The prepared Ad-PEG-MeAC (0.09 mg, 0.00007 mmol) was dissolved in an aqueous solution with EDC (1-ethyl-3-(3-dimethylaminopropyl)carbodiimide hydrochloride) (0.28 mg, 0.0015 mmol). rhlL-2 (1 mg) was dissolved in an aqueous solution of Ad-PEG-NH₂ + EDC. The reaction solution was purged in N₂ atmosphere for 10 min. The reaction was performed for 2 hr at room temperature. After reaction, purification was carried out with dialysis and lyophilization. A schematic illustration of the synthesis is shown in FIG. 27.

### (4) Characterization of β-CD PPZ/Ad-IL2

As a result of electrophoresis of a ladder, IL-2, and Ad-IL2 spreading on a polyacrylamide gel according to their molecular weight, the molecular weight of Ad-IL2 was found to be higher than that of IL-2.

### Experimental Example 7: Cancer Cell Growth-Inhibiting Activity of Conjugate of Example 3

### (1) Experimental method

5.0 × 10⁵ cells of B16 melanoma cell line was injected into B6 mice (0 day, n = 5). When the size of the cancer tissue reached 100 mm³ or larger, β-CD PPZ+Ad-IL2 complex and PBS alone were prepared and directly injected into the cancer tissues of each mouse, respectively. The size of the mouse cancer tissue was measured for a total of 12 days, and mean values thereof were calculated.

### (2) Experimental result

In the control PBS-injected group, it was confirmed that the size of the cancer tissue grew uncontrollably. During the experimental period, 2 mice out of 5 mice in the PBS group died due to hyperplasia of the cancer tissue. In contrast, in the group injected with β-CD PPZ+Ad-IL2 complex, all of the mice survived and the size of cancer tissue was also significantly well-suppressed, compared to the control group.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

### Effect of the invention

A hydrogel composition of the present disclosure is prepared by linking a physiologically active material to thermosensitive poly(phosphazene) via a host-guest interaction. When injected into a living body, the hydrogel composition slowly releases the physiologically active material and provides favorable conditions for stem cell differentiation. In addition, the hydrogel composition, in which the kind, ratio, and sequence of the physiologically active material are controlled to be suitable for stem cell differentiation, may be prepared with high reproducibility.

## Claims

1. A hydrogel inclusion complex comprising thermosensitive poly(phosphazene) to which a plurality of hydrophobic amino acids, hydrophilic polymers, and beta-cyclodextrin (β-cydodextrin; β-CD) as a host molecule are substituted; and
a physiologically active material linked directly or via a linker to one or more molecules, as a guest molecule, selected from the group consisting of adamantine, azobenzene, cholesterol, tert-butyl, cyclohexyl ester, and naphthyl,
wherein the guest molecule is conjugated to all or part of the beta-cyclodextrin by inclusion of the guest molecule into the beta-cyclodextrin via a host-guest interaction.

2. The hydrogel inclusion complex of claim 1, wherein the thermosensitive poly(phosphazene) includes a plurality of hydrophobic amino acids, hydrophilic polymers, and beta-cyclodextrin at a molar ratio of (55 to 80) : (5 to 25) : (5 to 20).

3. The hydrogel inclusion complex of claim 1, wherein the physiologically active material is any one or more selected from the group consisting of proteins, peptides, vaccines, genes, hormones, anti-cancer drugs, angiogenesis inhibitors, sugars, polyols, sugar-containing polyols, polymer-containing polyols, sugar-containing amino acids, and sugar-containing ions.

4. The hydrogel inclusion complex of claim 3, wherein the proteins are selected from the group consisting of exendin-4, erythropoietin (EPO), interferon-alpha, interferon-beta, interferon-gamma, growth hormone (human, pig, cow, etc.), growth hormone releasing factor, nerve growth factor (NGF), granulocyte-colony stimulating factor (G-CSF), granulocyte macrophage-colony stimulating factor (GM-CSF), macrophage-colony stimulating factor (M-CSF), blood clotting factor, insulin, oxytocin, vasopressin, adrenocorticotropic hormone, fibroblast growth factor (FGF), epidermal growth factor (EGF), platelet-derived growth factor (PDGF), insulin-like growth factor (IGF), vascular endothelial growth factor (VEGF), transforming growth factor-beta (TGF-β), nerve growth factor, brain-derived neurotrophic factor (BDNF), neurotrophin-3 (NT-3), neurotrophin-4/5, prolactin, luliberin, luteinizing hormone releasing hormone (LHRH), LHRH agonists, LHRH antagonists, somatostatin, glucagon, interleukin-2 (IL-2), interleukin-11 (IL-11), gastrin, tetragastrin, pentagastrin, urogastrone, secretin, calcitonin, enkephalins, endorphins, angiotensins, thyrotropin releasing hormone (TRH), tumor necrosis factor (TNF), tumor necrosis factor related apoptosis inducing ligand (TRAIL), heparinase, bone morphogenic protein (BMP), human atrial natriuretic peptide (hANP), glucagon-like peptide (GLP-1), renin, bradykinin, bacitracins, polymyxins, colistins, tyrocidine, gramicidins, cyclosporins, neurotensin, tachykinin, neuropeptide Y (NPY), peptide YY (PYY), vasoactive intestinal polypeptide (VIP), and pituitary adenylate cyclase-activating polypeptide (PACAP).

5. The hydrogel inclusion complex of claim 3, wherein the peptide is selected from the group consisting of collagen 1-derived GFOGER and DGEA; laminin-derived YIGSR, SIKVAV, IKVAV, IKLLI, LRGDN, and SINNNR; laminin γ1-derived LRE, PDGSR, GTFALRGDNGQ, CFALRGDNP, NPWHSIYITRFG, TWYKIAFQRNRK, KAFDITYVRLKF, and LGTIPG; fibronectin-derived GRGDS, PKRGDL, NGRAHA, GACRGDCLGA(cyclic), IDAPS, REDV, PHSRN, KQAGDV, LDV, WQPPRARI, SPPRRARV, LIGRKK, IWKHKGRDVILKKDVRFYC, KLDAPT, and PRARI; vitronectin-derived CKKQRFRHRNRKG; osteopontin-derived KRSR, FHRRIKA, CGGNGEPRGDTYRAY, SVVYGLR, and ELVTDFPTDLPAT; elastin-derived VPGIG and VGVAPG; collagen 4-derived MNYYSNS and CNYYSNS; thrombospondin-derived CSVTCG, GRGDAC, FQGVLQNVRFVF, AELDVP, and VALDEP; nidogen-1-derived GFRGDGQ and SIGFRGDGQTC; N-cadherin-derived HAV; and TGF-β1-derived FLPASGL, PWPLPYL, WGLLDLT, PAERLRS, RNLDGWS, NLSSSWI, TLPSNTH, MSAFPFL, SRLGQYI, PFGPLPP, TIASTLH, PRAPADV, and ESPLKRQ.

6. The hydrogel inclusion complex of claim 1, wherein the guest molecule and the physiologically active material are linked via a linker, which is polyethylene glycol (PEG), polyetherimide (PEI), or polypropylene glycol (PPG) having a molecular weight of 200 Da to 5,000 Da, or a polypeptide selected from the group consisting of polyglycine, polyhistidine, and poly(RADA).

7. A hydrogel of controlling stem cell differentiation, which comprises as active ingredients, thermosensitive poly(phosphazene) to which a plurality of hydrophobic amino acids, hydrophilic polymers, and beta-cyclodextrin are substituted; and a stem cell differentiation regulator linked directly or via a linker to one or more molecules, as a guest molecule, selected from the group consisting of adamantine, azobenzene, cholesterol, tert-butyl, cyclohexyl ester, and naphthyl.

8. The hydrogel of claim 7, wherein the guest molecule is conjugated to all or part of the beta-cyclodextrin by inclusion of the guest molecule into the beta-cyclodextrin via a host-guest interaction.

9. The hydrogel of claim 7, wherein the thermosensitive poly(phosphazene) to which a plurality of hydrophobic amino acids, hydrophilic polymers, and beta-cyclodextrin are substituted; and the stem cell differentiation regulator linked directly or via a linker to one or more molecules, as a guest molecule, selected from the group consisting of adamantine, azobenzene, cholesterol, tert-butyl, cyclohexyl ester, and naphthyl are provided independently or in the form of a complex which is formed by forming a conjugate by inclusion of the guest molecule in the beta-cyclodextrin via a host-guest interaction.

10. The hydrogel of claim 7, wherein the stem cells are controlled to be differentiated to a specific state by controlling the type or ratio of the stem cell differentiation regulator, or by controlling both of them.

11. The hydrogel of claim 7, wherein the stem cell differentiation regulator is a peptide comprising arginine-lysine-aspartic acid (RGD).

12. The hydrogel of claim 7, wherein the stem cell differentiation regulator is a peptide comprising CESPLKRQ and a peptide comprising CLRAHAVDIN.

13. A method of regenerating a tissue, the method comprising a step of injecting, into a damaged tissue site, a hydrogel composition comprising, as active ingredients, thermosensitive poly(phosphazene) to which a plurality of hydrophobic amino acids, hydrophilic polymers, and beta-cyclodextrin are substituted; and a stem cell differentiation regulator linked directly or via a linker to one or more molecules, as a guest molecule, selected from the group consisting of adamantine, azobenzene, cholesterol, tert-butyl, cyclohexyl ester, and naphthyl.

14. The method of claim 13, wherein the hydrogel composition further comprises stem cells.

15. The method of claim 13, wherein the hydrogel composition is introduced by injection.

16. A hydrogel of inhibiting cancer cell proliferation or metastasis, which comprises as active ingredients, thermosensitive poly(phosphazene) to which a plurality of hydrophobic amino acids, hydrophilic polymers, and beta-cyclodextrin are substituted; and IL-2 linked directly or via a linker to one or more molecules, as a guest molecule, selected from the group consisting of adamantine, azobenzene, cholesterol, tert-butyl, cyclohexyl ester, and naphthyl.

17. Thermosensitive poly(phosphazene) to which a plurality of hydrophobic amino acids, hydrophilic polymers, and beta-cyclodextrin are substituted.

18. The thermosensitive poly(phosphazene) of claim 17, wherein the beta-cyclodextrin is linked to the main chain of poly(phosphazene) via a hydroxyl group at C6 position of C₁₋₆ alkylene diamine, poly(C₁₋₆ alkylene diamine), n-amino-n-oxoalkanoic acid (wherein n is an integer of 2 to 6), thiol, carboxylate, C₂₋₆ hydroxyalkyl m-amino-m-oxoalkanoic acid (wherein m is an integer of 2 to 6), or cyano-amino-C₁₋₄ alkylthio-C₁₋₆ alkane as a linker.
